# EUROPEAN PATENT APPLICATION

(11) **EP 2 003 200 A1**
(43) Date of publication of application: **17.12.2008**
(21) Application number: 07740017.4
(22) Date of filing: 28.03.2007
(51) Int. Cl.: C12N 15/09, C07K 14/005, C12N 1/21, C12N 7/00, C12Q 1/70, C40B 40/06, C40B 40/10

(54) **PHAGE DISPLAY BY NOVEL FILAMENTOUS BACTERIOPHAGE**

(30) Priority: 06.04.2006 JP 2006105143
(71) Applicant: Uchiyama, Fumiaki, Jonan-ku Fukuoka-shi Fukuoka 814-0105 (JP)
(72) Inventor: Uchiyama, Fumiaki, Jonan-ku Fukuoka-shi Fukuoka 814-0105 (JP)
(74) Representative: Friede, Thomas
(86) International application number: PCT/JP2007/056579
(87) International publication number: WO 2007/114139

(57) **Abstract**

[PROBLEMS] To provide a phage display vector which can be fused in the inside of pIII and a phage display method using the vector. [MEANS FOR SOLVING PROBLEMS] Disclosed is a phage display method which is **characterized by** using a mutant pIII protein having an amino acid residue inserted between a proline residue at position 11 and a histidine residue at position 12 in an M13 phage pIII protein as depicted in SEQ ID NO: 1. The method enables to produce a random peptide with high efficiency and to provide a novel source in peptide conformation.

SEQUENCE LISTING

<110>  Uchiyama, Fumiaki

<120>  Pharge display using novel filamentous bacteriophage

<130>  PU5-9001WO

<160>  38

<170>  PatentIn version 3.1

<210>  1
<211>  406
<212>  PRT
<213>  filamentous bacteriophage

<400>  1

Ala Glu Thr Val Glu Ser Cys Leu Ala Lys Pro His Thr Glu Asn Ser
1               5                   10                  15

Phe Thr Asn Val Trp Lys Asp Asp Lys Thr Leu Asp Arg Tyr Ala Asn
            20                  25                  30

Tyr Glu Gly Cys Leu Trp Asn Ala Thr Gly Val Val Val Cys Thr Gly
        35                  40                  45

Asp Glu Thr Gln Cys Tyr Gly Thr Trp Val Pro Ile Gly Leu Ala Ile
    50                  55                  60

Pro Glu Asn Glu Gly Gly Gly Ser Glu Gly Gly Gly Ser Glu Gly Gly
65                  70                  75                  80

Gly Ser Glu Gly Gly Gly Thr Lys Pro Pro Glu Tyr Gly Asp Thr Pro
                85                  90                  95

Ile Pro Gly Tyr Thr Tyr Ile Asn Pro Leu Asp Gly Thr Tyr Pro Pro
            100                 105                 110

Gly Thr Glu Gln Asn Pro Ala Asn Pro Asn Pro Ser Leu Glu Glu Ser
        115                 120                 125

Gln Pro Leu Asn Thr Phe Met Phe Gln Asn Asn Arg Phe Arg Asn Arg
    130                 135                 140

Gln Gly Ala Leu Thr Val Tyr Thr Gly Thr Val Thr Gln Gly Thr Asp
145                 150                 155                 160

Pro Val Lys Thr Tyr Tyr Gln Tyr Thr Pro Val Ser Ser Lys Ala Met
                165                 170                 175

Tyr Asp Ala Tyr Trp Asn Gly Lys Phe Arg Asp Cys Ala Phe His Ser
            180                 185                 190

Gly Phe Asn Glu Asp Pro Phe Val Cys Glu Tyr Gln Gly Gln Ser Ser

195                 200                 205

Asp Leu Pro Gln Pro Pro Val Asn Ala Gly Gly Gly Ser Gly Gly Gly
    210                 215                 220

Ser Gly Gly Gly Ser Glu Gly Gly Gly Ser Glu Gly Gly Gly Ser Glu
225                 230                 235                 240

Gly Gly Gly Ser Glu Gly Gly Gly Ser Gly Gly Gly Ser Gly Ser Gly
                245                 250                 255

Asp Phe Asp Tyr Glu Lys Met Ala Asn Ala Asn Lys Gly Ala Met Thr
            260                 265                 270

Glu Asn Ala Asp Glu Asn Ala Leu Gln Ser Asp Ala Lys Gly Lys Leu
        275                 280                 285

Asp Ser Val Ala Thr Asp Tyr Gly Ala Ala Ile Asp Gly Phe Ile Gly
    290                 295                 300

Asp Val Ser Gly Leu Ala Asn Gly Asn Gly Ala Thr Gly Asp Phe Ala
305                 310                 315                 320

Gly Ser Asn Ser Gln Met Ala Gln Val Gly Asp Gly Asp Asn Ser Pro
                325                 330                 335

Leu Met Asn Asn Phe Arg Gln Tyr Leu Pro Ser Leu Pro Gln Ser Val
            340                 345                 350

Glu Cys Arg Pro Phe Val Phe Ser Ala Gly Lys Pro Tyr Glu Phe Ser
        355                 360                 365

Ile Asp Cys Asp Lys Ile Asn Leu Phe Arg Gly Val Phe Ala Phe Leu
    370                 375                 380

Leu Tyr Val Ala Thr Phe Met Tyr Val Phe Ser Thr Phe Ala Asn Ile
385                 390                 395                 400

Leu Arg Asn Lys Glu Ser
                405

<210>  2
<211>  410
<212>  PRT
<213>  filamentous bacteriophage

<400>  2

Ala Glu Thr Val Glu Ser Cys Leu Ala Lys Pro Asp Ile Gly Thr His
1               5                   10                  15

Thr Glu Asn Ser Phe Thr Asn Val Trp Lys Asp Asp Lys Thr Leu Asp
            20                  25                  30

Arg Tyr Ala Asn Tyr Glu Gly Cys Leu Trp Asn Ala Thr Gly Val Val
        35                  40                  45

Val Cys Thr Gly Asp Glu Thr Gln Cys Tyr Gly Thr Trp Val Pro Ile
    50                  55                  60

Gly Leu Ala Ile Pro Glu Asn Glu Gly Gly Gly Ser Glu Gly Gly Gly
65                  70                  75                  80

Ser Glu Gly Gly Gly Ser Glu Gly Gly Gly Thr Lys Pro Pro Glu Tyr
                85                  90                  95

Gly Asp Thr Pro Ile Pro Gly Tyr Thr Tyr Ile Asn Pro Leu Asp Gly
            100                 105                 110

Thr Tyr Pro Pro Gly Thr Glu Gln Asn Pro Ala Asn Pro Asn Pro Ser
        115                 120                 125

Leu Glu Glu Ser Gln Pro Leu Asn Thr Phe Met Phe Gln Asn Asn Arg
    130                 135                 140

Phe Arg Asn Arg Gln Gly Ala Leu Thr Val Tyr Thr Gly Thr Val Thr
145                 150                 155                 160

Gln Gly Thr Asp Pro Val Lys Thr Tyr Tyr Gln Tyr Thr Pro Val Ser
                165                 170                 175

Ser Lys Ala Met Tyr Asp Ala Tyr Trp Asn Gly Lys Phe Arg Asp Cys
            180                 185                 190

Ala Phe His Ser Gly Phe Asn Glu Asp Pro Phe Val Cys Glu Tyr Gln
        195                 200                 205

Gly Gln Ser Ser Asp Leu Pro Gln Pro Pro Val Asn Ala Gly Gly Gly
    210                 215                 220

Ser Gly Gly Gly Ser Gly Gly Gly Ser Glu Gly Gly Gly Ser Glu Gly
225                 230                 235                 240

Gly Gly Ser Glu Gly Gly Gly Ser Glu Gly Gly Gly Ser Gly Gly Gly
                245                 250                 255

Ser Gly Ser Gly Asp Phe Asp Tyr Glu Lys Met Ala Asn Ala Asn Lys
            260                 265                 270

Gly Ala Met Thr Glu Asn Ala Asp Glu Asn Ala Leu Gln Ser Asp Ala
        275                 280                 285

Lys Gly Lys Leu Asp Ser Val Ala Thr Asp Tyr Gly Ala Ala Ile Asp
    290                 295                 300

Gly Phe Ile Gly Asp Val Ser Gly Leu Ala Asn Gly Asn Gly Ala Thr
305                 310                 315                 320

Gly Asp Phe Ala Gly Ser Asn Ser Gln Met Ala Gln Val Gly Asp Gly
                325                 330                 335

Asp Asn Ser Pro Leu Met Asn Asn Phe Arg Gln Tyr Leu Pro Ser Leu
            340                 345                 350

Pro Gln Ser Val Glu Cys Arg Pro Phe Val Phe Ser Ala Gly Lys Pro
        355                 360                 365

Tyr Glu Phe Ser Ile Asp Cys Asp Lys Ile Asn Leu Phe Arg Gly Val
    370                 375                 380

Phe Ala Phe Leu Leu Tyr Val Ala Thr Phe Met Tyr Val Phe Ser Thr
385                 390                 395                 400

Phe Ala Asn Ile Leu Arg Asn Lys Glu Ser
                405                 410

<210>  3
<211>  421
<212>  PRT
<213>  filamentous bacteriophage

<400>  3

Ala Glu Thr Val Glu Ser Cys Leu Ala Lys Pro Asp Ile Asp His Lys
1               5                   10                  15

Ser Gly Ser Arg Asp Gln Leu Ser Gly Thr His Thr Glu Asn Ser Phe
            20                  25                  30

Thr Asn Val Trp Lys Asp Asp Lys Thr Leu Asp Arg Tyr Ala Asn Tyr
        35                  40                  45

Glu Gly Cys Leu Trp Asn Ala Thr Gly Val Val Val Cys Thr Gly Asp
    50                  55                  60

Glu Thr Gln Cys Tyr Gly Thr Trp Val Pro Ile Gly Leu Ala Ile Pro
65                  70                  75                  80

Glu Asn Glu Gly Gly Gly Ser Glu Gly Gly Gly Ser Glu Gly Gly Gly
                85                  90                  95

Ser Glu Gly Gly Gly Thr Lys Pro Pro Glu Tyr Gly Asp Thr Pro Ile
            100                 105                 110

Pro Gly Tyr Thr Tyr Ile Asn Pro Leu Asp Gly Thr Tyr Pro Pro Gly
        115                 120                 125

Thr Glu Gln Asn Pro Ala Asn Pro Asn Pro Ser Leu Glu Glu Ser Gln
    130                 135                 140

Pro Leu Asn Thr Phe Met Phe Gln Asn Asn Arg Phe Arg Asn Arg Gln
145                 150                 155                 160

Gly Ala Leu Thr Val Tyr Thr Gly Thr Val Thr Gln Gly Thr Asp Pro
                165                 170                 175

Val Lys Thr Tyr Tyr Gln Tyr Thr Pro Val Ser Ser Lys Ala Met Tyr
            180                 185                 190

Asp Ala Tyr Trp Asn Gly Lys Phe Arg Asp Cys Ala Phe His Ser Gly
        195                 200                 205

Phe Asn Glu Asp Pro Phe Val Cys Glu Tyr Gln Gly Gln Ser Ser Asp
    210                 215                 220

Leu Pro Gln Pro Pro Val Asn Ala Gly Gly Gly Ser Gly Gly Gly Ser
225                 230                 235                 240

Gly Gly Gly Ser Glu Gly Gly Gly Ser Glu Gly Gly Gly Ser Glu Gly
                245                 250                 255

Gly Gly Ser Glu Gly Gly Gly Ser Gly Gly Gly Ser Gly Ser Gly Asp
            260                 265                 270

Phe Asp Tyr Glu Lys Met Ala Asn Ala Asn Lys Gly Ala Met Thr Glu
        275                 280                 285

Asn Ala Asp Glu Asn Ala Leu Gln Ser Asp Ala Lys Gly Lys Leu Asp
    290                 295                 300

Ser Val Ala Thr Asp Tyr Gly Ala Ala Ile Asp Gly Phe Ile Gly Asp
305                 310                 315                 320

Val Ser Gly Leu Ala Asn Gly Asn Gly Ala Thr Gly Asp Phe Ala Gly
                325                 330                 335

Ser Asn Ser Gln Met Ala Gln Val Gly Asp Gly Asp Asn Ser Pro Leu
            340                 345                 350

Met Asn Asn Phe Arg Gln Tyr Leu Pro Ser Leu Pro Gln Ser Val Glu
        355                 360                 365

Cys Arg Pro Phe Val Phe Ser Ala Gly Lys Pro Tyr Glu Phe Ser Ile
    370                 375                 380

Asp Cys Asp Lys Ile Asn Leu Phe Arg Gly Val Phe Ala Phe Leu Leu
385                 390                 395                 400

Tyr Val Ala Thr Phe Met Tyr Val Phe Ser Thr Phe Ala Asn Ile Leu
                405                 410                 415

Arg Asn Lys Glu Ser
            420

<210>  4
<211>  427
<212>  PRT
<213>  filamentous bacteriophage

<400>  4

Ala Glu Thr Val Glu Ser Cys Leu Ala Lys Pro Asp Gly Ser Ala Gly
1               5                   10                  15

Cys Lys Asn Phe Phe Trp Lys Thr Phe Thr Ser Cys Asp Ile Gly Thr
            20                  25                  30

His Thr Glu Asn Ser Phe Thr Asn Val Trp Lys Asp Asp Lys Thr Leu
        35                  40                  45

Asp Arg Tyr Ala Asn Tyr Glu Gly Cys Leu Trp Asn Ala Thr Gly Val
    50                  55                  60

Val Val Cys Thr Gly Asp Glu Thr Gln Cys Tyr Gly Thr Trp Val Pro
65                  70                  75                  80

Ile Gly Leu Ala Ile Pro Glu Asn Glu Gly Gly Gly Ser Glu Gly Gly
                85                  90                  95

Gly Ser Glu Gly Gly Gly Ser Glu Gly Gly Gly Thr Lys Pro Pro Glu
            100                 105                 110

Tyr Gly Asp Thr Pro Ile Pro Gly Tyr Thr Tyr Ile Asn Pro Leu Asp
        115                 120                 125

Gly Thr Tyr Pro Pro Gly Thr Glu Gln Asn Pro Ala Asn Pro Asn Pro
    130                 135                 140

Ser Leu Glu Glu Ser Gln Pro Leu Asn Thr Phe Met Phe Gln Asn Asn
145                 150                 155                 160

Arg Phe Arg Asn Arg Gln Gly Ala Leu Thr Val Tyr Thr Gly Thr Val
                165                 170                 175

Thr Gln Gly Thr Asp Pro Val Lys Thr Tyr Tyr Gln Tyr Thr Pro Val
            180                 185                 190

Ser Ser Lys Ala Met Tyr Asp Ala Tyr Trp Asn Gly Lys Phe Arg Asp
        195                 200                 205

Cys Ala Phe His Ser Gly Phe Asn Glu Asp Pro Phe Val Cys Glu Tyr
    210                 215                 220

Gln Gly Gln Ser Ser Asp Leu Pro Gln Pro Pro Val Asn Ala Gly Gly
225                 230                 235                 240

Gly Ser Gly Gly Gly Ser Gly Gly Gly Ser Glu Gly Gly Gly Ser Glu
                245                 250                 255

Gly Gly Gly Ser Glu Gly Gly Gly Ser Glu Gly Gly Gly Ser Gly Gly
            260                 265                 270

Gly Ser Gly Ser Gly Asp Phe Asp Tyr Glu Lys Met Ala Asn Ala Asn
        275                 280                 285

Lys Gly Ala Met Thr Glu Asn Ala Asp Glu Asn Ala Leu Gln Ser Asp
    290                 295                 300

Ala Lys Gly Lys Leu Asp Ser Val Ala Thr Asp Tyr Gly Ala Ala Ile
305                 310                 315                 320

Asp Gly Phe Ile Gly Asp Val Ser Gly Leu Ala Asn Gly Asn Gly Ala
                325                 330                 335

Thr Gly Asp Phe Ala Gly Ser Asn Ser Gln Met Ala Gln Val Gly Asp
            340                 345                 350

Gly Asp Asn Ser Pro Leu Met Asn Asn Phe Arg Gln Tyr Leu Pro Ser
        355                 360                 365

Leu Pro Gln Ser Val Glu Cys Arg Pro Phe Val Phe Ser Ala Gly Lys
    370                 375                 380

Pro Tyr Glu Phe Ser Ile Asp Cys Asp Lys Ile Asn Leu Phe Arg Gly
385                 390                 395                 400

Val Phe Ala Phe Leu Leu Tyr Val Ala Thr Phe Met Tyr Val Phe Ser
                405                 410                 415

Thr Phe Ala Asn Ile Leu Arg Asn Lys Glu Ser
            420                 425

<210> 5
<211> 430
<212> PRT
<213> filamentous bacteriophage

<400> 5

Ala Glu Thr Val Glu Ser Cys Leu Ala Lys Pro Asp Gly Ser Ala Gly
1               5                   10                  15

Cys Lys Asn Phe Phe Trp Lys Thr Phe Thr Ser Cys Ile Glu Gly Arg

            20                  25                  30

Thr Gly Thr His Thr Glu Asn Ser Phe Thr Asn Val Trp Lys Asp Asp
        35                  40                  45

Lys Thr Leu Asp Arg Tyr Ala Asn Tyr Glu Gly Cys Leu Trp Asn Ala
    50                  55                  60

Thr Gly Val Val Val Cys Thr Gly Asp Glu Thr Gln Cys Tyr Gly Thr
65                  70                  75                  80

Trp Val Pro Ile Gly Leu Ala Ile Pro Glu Asn Glu Gly Gly Gly Ser
                85                  90                  95

Glu Gly Gly Gly Ser Glu Gly Gly Gly Ser Glu Gly Gly Gly Thr Lys
            100                 105                 110

Pro Pro Glu Tyr Gly Asp Thr Pro Ile Pro Gly Tyr Thr Tyr Ile Asn
        115                 120                 125

Pro Leu Asp Gly Thr Tyr Pro Pro Gly Thr Glu Gln Asn Pro Ala Asn
    130                 135                 140

Pro Asn Pro Ser Leu Glu Glu Ser Gln Pro Leu Asn Thr Phe Met Phe
145                 150                 155                 160

Gln Asn Asn Arg Phe Arg Asn Arg Gln Gly Ala Leu Thr Val Tyr Thr
                165                 170                 175

Gly Thr Val Thr Gln Gly Thr Asp Pro Val Lys Thr Tyr Tyr Gln Tyr
            180                 185                 190

Thr Pro Val Ser Ser Lys Ala Met Tyr Asp Ala Tyr Trp Asn Gly Lys
        195                 200                 205

Phe Arg Asp Cys Ala Phe His Ser Gly Phe Asn Glu Asp Pro Phe Val
    210                 215                 220

Cys Glu Tyr Gln Gly Gln Ser Ser Asp Leu Pro Gln Pro Pro Val Asn
225                 230                 235                 240

Ala Gly Gly Gly Ser Gly Gly Gly Ser Gly Gly Gly Ser Glu Gly Gly
                245                 250                 255

Gly Ser Glu Gly Gly Gly Ser Glu Gly Gly Gly Ser Glu Gly Gly Gly
            260                 265                 270

Ser Gly Gly Gly Ser Gly Ser Gly Asp Phe Asp Tyr Glu Lys Met Ala
        275                 280                 285

Asn Ala Asn Lys Gly Ala Met Thr Glu Asn Ala Asp Glu Asn Ala Leu

290                 295                 300

Gln Ser Asp Ala Lys Gly Lys Leu Asp Ser Val Ala Thr Asp Tyr Gly
305                 310                 315                 320

Ala Ala Ile Asp Gly Phe Ile Gly Asp Val Ser Gly Leu Ala Asn Gly
                325                 330                 335

Asn Gly Ala Thr Gly Asp Phe Ala Gly Ser Asn Ser Gln Met Ala Gln
            340                 345                 350

Val Gly Asp Gly Asp Asn Ser Pro Leu Met Asn Asn Phe Arg Gln Tyr
        355                 360                 365

Leu Pro Ser Leu Pro Gln Ser Val Glu Cys Arg Pro Phe Val Phe Ser
    370                 375                 380

Ala Gly Lys Pro Tyr Glu Phe Ser Ile Asp Cys Asp Lys Ile Asn Leu
385                 390                 395                 400

Phe Arg Gly Val Phe Ala Phe Leu Leu Tyr Val Ala Thr Phe Met Tyr
                405                 410                 415

Val Phe Ser Thr Phe Ala Asn Ile Leu Arg Asn Lys Glu Ser
            420                 425                 430

<210>  6
<211>  431
<212>  PRT
<213>  filamentous bacteriophage

<220>
<221>  MISC_FEATURE
<223>  X is any amino acid

<220>
<221>  MISC_FEATURE
<222>  (21)..(24)
<223>  X is any amino acid

<400>  6

Ala Glu Thr Val Glu Ser Cys Leu Ala Lys Pro Asp Ile Asp His Lys
1               5                   10                  15

Cys Lys Asn Phe Xaa Xaa Xaa Xaa Phe Thr Ser Cys Ile Glu Gly Arg
            20                  25                  30

Leu Ser Gly Thr His Thr Glu Asn Ser Phe Thr Asn Val Trp Lys Asp
        35                  40                  45

Asp Lys Thr Leu Asp Arg Tyr Ala Asn Tyr Glu Gly Cys Leu Trp Asn
    50                  55                  60

Ala Thr Gly Val Val Val Cys Thr Gly Asp Glu Thr Gln Cys Tyr Gly
65                  70                  75                  80

Thr Trp Val Pro Ile Gly Leu Ala Ile Pro Glu Asn Glu Gly Gly Gly
                85                  90                  95

Ser Glu Gly Gly Gly Ser Glu Gly Gly Gly Ser Glu Gly Gly Gly Thr
            100                 105                 110

Lys Pro Pro Glu Tyr Gly Asp Thr Pro Ile Pro Gly Tyr Thr Tyr Ile
        115                 120                 125

Asn Pro Leu Asp Gly Thr Tyr Pro Pro Gly Thr Glu Gln Asn Pro Ala
    130                 135                 140

Asn Pro Asn Pro Ser Leu Glu Glu Ser Gln Pro Leu Asn Thr Phe Met
145                 150                 155                 160

Phe Gln Asn Asn Arg Phe Arg Asn Arg Gln Gly Ala Leu Thr Val Tyr
                165                 170                 175

Thr Gly Thr Val Thr Gln Gly Thr Asp Pro Val Lys Thr Tyr Tyr Gln
            180                 185                 190

Tyr Thr Pro Val Ser Ser Lys Ala Met Tyr Asp Ala Tyr Trp Asn Gly
        195                 200                 205

Lys Phe Arg Asp Cys Ala Phe His Ser Gly Phe Asn Glu Asp Pro Phe
    210                 215                 220

Val Cys Glu Tyr Gln Gly Gln Ser Ser Asp Leu Pro Gln Pro Pro Val
225                 230                 235                 240

Asn Ala Gly Gly Gly Ser Gly Gly Gly Ser Gly Gly Gly Ser Glu Gly
                245                 250                 255

Gly Gly Ser Glu Gly Gly Gly Ser Glu Gly Gly Gly Ser Glu Gly Gly
            260                 265                 270

Gly Ser Gly Gly Gly Ser Gly Ser Gly Asp Phe Asp Tyr Glu Lys Met
        275                 280                 285

Ala Asn Ala Asn Lys Gly Ala Met Thr Glu Asn Ala Asp Glu Asn Ala
    290                 295                 300

Leu Gln Ser Asp Ala Lys Gly Lys Leu Asp Ser Val Ala Thr Asp Tyr
305                 310                 315                 320

Gly Ala Ala Ile Asp Gly Phe Ile Gly Asp Val Ser Gly Leu Ala Asn
                325                 330                 335

Gly Asn Gly Ala Thr Gly Asp Phe Ala Gly Ser Asn Ser Gln Met Ala
            340                 345                 350

Gln Val Gly Asp Gly Asp Asn Ser Pro Leu Met Asn Asn Phe Arg Gln
        355                 360                 365

Tyr Leu Pro Ser Leu Pro Gln Ser Val Glu Cys Arg Pro Phe Val Phe
    370                 375                 380

Ser Ala Gly Lys Pro Tyr Glu Phe Ser Ile Asp Cys Asp Lys Ile Asn
385                 390                 395                 400

Leu Phe Arg Gly Val Phe Ala Phe Leu Leu Tyr Val Ala Thr Phe Met
                405                 410                 415

Tyr Val Phe Ser Thr Phe Ala Asn Ile Leu Arg Asn Lys Glu Ser
            420                 425                 430

<210>  7
<211>  1296
<212>  DNA
<213>  filamentous bacteriophage

<220>
<221>  misc_feature
<222>  (61)..(72)
<223>  n is any base

<400>  7
gctgaaactg ttgaaagttg tttagcaaaa cccgatatcg accataagtg taagaacttc      60

nnnnnnnnnn nnttcacatc ttgcattgaa ggtagattgt ctggtaccca tacagaaaat     120

tcatttacta acgtctggaa agacgacaaa actttagatc gttacgctaa ctatgagggt     180

tgtctgtgga atgctacagg cgttgtagtt tgtactggtg acgaaactca gtgttacggt     240

acatgggttc ctattgggct tgctatccct gaaaatgagg gtggtggctc tgagggtggc     300

ggttctgagg gtggcggttc tgagggtggc ggtactaaac ctcctgagta cggtgataca     360

cctattccgg gctatactta tatcaaccct ctcgacggca cttatccgcc tggtactgag     420

caaaaccccg ctaatcctaa tccttctctt gaggagtctc agcctcttaa tactttcatg     480

tttcagaata ataggttccg aaataggcag ggggcattaa ctgtttatac gggcactgtt     540

actcaaggca ctgaccccgt taaaacttat taccagtaca ctcctgtatc atcaaaagcc     600

atgtatgacg cttactggaa cggtaaattc agagactgcg ctttccattc tggctttaat     660

gaggatccat tcgtttgtga atatcaaggc caatcgtctg acctgcctca acctcctgtc     720

aatgctggcg gcggctctgg tggtggttct ggtggcggct ctgagggtgg tggctctgag     780

ggtggcggtt ctgagggtgg cggctctgag ggaggcggtt ccggtggtgg ctctggttcc     840

ggtgattttg attatgaaaa gatggcaaac gctaataagg gggctatgac cgaaaatgcc     900

gatgaaaacg cgctacagtc tgacgctaaa ggcaaacttg attctgtcgc tactgattac     960

ggtgctgcta tcgatggttt cattggtgac gtttccggcc ttgctaatgg taatggtgct 1020

actggtgatt ttgctggctc taattcccaa atggctcaag tcggtgacgg tgataattca 1080

cctttaatga ataatttccg tcaatattta ccttccctcc ctcaatcggt tgaatgtcgc 1140

ccttttgtct ttagcgctgg taaaccatat gaattttcta ttgattgtga caaaataaac 1200

ttattccgtg gtgtctttgc gtttctttta tatgttgcca cctttatgta tgtattttct 1260

acgtttgcta acatactgcg taataaggag tcttaa 1296

<210> 8
<211> 433
<212> PRT
<213> filamentous bacteriophage

<220>
<221> MISC_FEATURE
<223> X is any amino acid

<220>
<221> MISC_FEATURE
<222> (19)..(32)
<223> X is any amino acid

<400> 8

Ala Glu Thr Val Glu Ser Cys Leu Ala Lys Pro Asp Ile Asp His Lys
1               5                   10                  15

Ser Gly Xaa Xaa Xaa Xaa Cys Xaa Xaa Xaa Xaa Cys Xaa Xaa Xaa Xaa
            20                  25                  30

Asp Gln Leu Ser Gly Thr His Thr Glu Asn Ser Phe Thr Asn Val Trp
        35                  40                  45

Lys Asp Asp Lys Thr Leu Asp Arg Tyr Ala Asn Tyr Glu Gly Cys Leu
    50                  55                  60

Trp Asn Ala Thr Gly Val Val Val Cys Thr Gly Asp Glu Thr Gln Cys
65                  70                  75                  80

Tyr Gly Thr Trp Val Pro Ile Gly Leu Ala Ile Pro Glu Asn Glu Gly
                85                  90                  95

Gly Gly Ser Glu Gly Gly Gly Ser Glu Gly Gly Gly Ser Glu Gly Gly
            100                 105                 110

Gly Thr Lys Pro Pro Glu Tyr Gly Asp Thr Pro Ile Pro Gly Tyr Thr
        115                 120                 125

Tyr Ile Asn Pro Leu Asp Gly Thr Tyr Pro Pro Gly Thr Glu Gln Asn
    130                 135                 140

Pro Ala Asn Pro Asn Pro Ser Leu Glu Glu Ser Gln Pro Leu Asn Thr
145                 150                 155                 160

Phe Met Phe Gln Asn Asn Arg Phe Arg Asn Arg Gln Gly Ala Leu Thr
                165                 170                 175

Val Tyr Thr Gly Thr Val Thr Gln Gly Thr Asp Pro Val Lys Thr Tyr
            180                 185                 190

Tyr Gln Tyr Thr Pro Val Ser Ser Lys Ala Met Tyr Asp Ala Tyr Trp
        195                 200                 205

Asn Gly Lys Phe Arg Asp Cys Ala Phe His Ser Gly Phe Asn Glu Asp
    210                 215                 220

Pro Phe Val Cys Glu Tyr Gln Gly Gln Ser Ser Asp Leu Pro Gln Pro
225                 230                 235                 240

Pro Val Asn Ala Gly Gly Gly Ser Gly Gly Gly Ser Gly Gly Gly Ser
                245                 250                 255

Glu Gly Gly Gly Ser Glu Gly Gly Gly Ser Glu Gly Gly Gly Ser Glu
            260                 265                 270

Gly Gly Gly Ser Gly Gly Gly Ser Gly Ser Gly Asp Phe Asp Tyr Glu
        275                 280                 285

Lys Met Ala Asn Ala Asn Lys Gly Ala Met Thr Glu Asn Ala Asp Glu
    290                 295                 300

Asn Ala Leu Gln Ser Asp Ala Lys Gly Lys Leu Asp Ser Val Ala Thr
305                 310                 315                 320

Asp Tyr Gly Ala Ala Ile Asp Gly Phe Ile Gly Asp Val Ser Gly Leu
                325                 330                 335

Ala Asn Gly Asn Gly Ala Thr Gly Asp Phe Ala Gly Ser Asn Ser Gln
            340                 345                 350

Met Ala Gln Val Gly Asp Gly Asp Asn Ser Pro Leu Met Asn Asn Phe
        355                 360                 365

Arg Gln Tyr Leu Pro Ser Leu Pro Gln Ser Val Glu Cys Arg Pro Phe
    370                 375                 380

Val Phe Ser Ala Gly Lys Pro Tyr Glu Phe Ser Ile Asp Cys Asp Lys
385                 390                 395                 400

Ile Asn Leu Phe Arg Gly Val Phe Ala Phe Leu Leu Tyr Val Ala Thr
                405                 410                 415

Phe Met Tyr Val Phe Ser Thr Phe Ala Asn Ile Leu Arg Asn Lys Glu
            420                 425                 430

Ser

<210>  9
<211>  1302
<212>  DNA
<213>  filamentous bacteriophage

<220>
<221>  misc_feature
<223>  n is any base

<220>
<221>  misc_feature
<222>  (55)..(96)
<223>  n is any base

<400>  9
gctgaaactg ttgaaagttg tttagcaaaa cccgatatcg accataagtc tggannnnnn      60

nnnnnntgcn nnnnnnnnnn ntgcnnnnnn nnnnnngacc aattgtctgg tacccataca     120

gaaaattcat ttactaacgt ctggaaagac gacaaaactt tagatcgtta cgctaactat     180

gagggttgtc tgtggaatgc tacaggcgtt gtagtttgta ctggtgacga aactcagtgt     240

tacggtacat gggttcctat tgggcttgct atccctgaaa atgagggtgg tggctctgag     300

ggtggcggtt ctgagggtgg cggttctgag ggtggcggta ctaaacctcc tgagtacggt     360

gatacaccta ttccgggcta tacttatatc aaccctctcg acggcactta tccgcctggt     420

actgagcaaa accccgctaa tcctaatcct tctcttgagg agtctcagcc tcttaatact     480

ttcatgtttc agaataatag gttccgaaat aggcaggggg cattaactgt ttatacgggc     540

actgttactc aaggcactga ccccgttaaa acttattacc agtacactcc tgtatcatca     600

aaagccatgt atgacgctta ctggaacggt aaattcagag actgcgcttt ccattctggc     660

tttaatgagg atccattcgt ttgtgaatat caaggccaat cgtctgacct gcctcaacct     720

cctgtcaatg ctggcggcgg ctctggtggt ggttctggtg gcggctctga gggtggtggc     780

tctgagggtg gcggttctga gggtggcggc tctgagggag gcggttccgg tggtggctct     840

ggttccggtg attttgatta tgaaaagatg gcaaacgcta ataagggggc tatgaccgaa     900

aatgccgatg aaaacgcgct acagtctgac gctaaaggca aacttgattc tgtcgctact     960

gattacggtg ctgctatcga tggtttcatt ggtgacgttt ccggccttgc taatggtaat    1020

ggtgctactg gtgattttgc tggctctaat tcccaaatgg ctcaagtcgg tgacggtgat    1080

aattcacctt taatgaataa tttccgtcaa tatttacctt ccctccctca atcggttgaa    1140

tgtcgccctt ttgtctttag cgctggtaaa ccatatgaat tttctattga ttgtgacaaa    1200

ataaacttat tccgtggtgt ctttgcgttt cttttatatg ttgccacctt tatgtatgta    1260

ttttctacgt ttgctaacat actgcgtaat aaggagtctt aa                        1302

<210>  10
<211>  436
<212>  PRT
<213>  filamentous bacteriophage

<220>
<221>  MISC_FEATURE
<223>  X is any amino acid

<220>
<221>  MISC_FEATURE
<222>  (19)..(30)
<223>  X is any amino acid

<400>  10

Ala Glu Thr Val Glu Ser Cys Leu Ala Lys Pro Asp Ile Asp His Lys
1               5                   10                  15

Ser Gly Xaa Cys Xaa Xaa Xaa Xaa Xaa Xaa Xaa Xaa Xaa Xaa Cys Ile
            20                  25                  30

Glu Gly Arg Leu Gln Leu Ser Gly Thr His Thr Glu Asn Ser Phe Thr
        35                  40                  45

Asn Val Trp Lys Asp Asp Lys Thr Leu Asp Arg Tyr Ala Asn Tyr Glu
    50                  55                  60

Gly Cys Leu Trp Asn Ala Thr Gly Val Val Val Cys Thr Gly Asp Glu
65                  70                  75                  80

Thr Gln Cys Tyr Gly Thr Trp Val Pro Ile Gly Leu Ala Ile Pro Glu
                85                  90                  95

Asn Glu Gly Gly Gly Ser Glu Gly Gly Gly Ser Glu Gly Gly Gly Ser
            100                 105                 110

Glu Gly Gly Gly Thr Lys Pro Pro Glu Tyr Gly Asp Thr Pro Ile Pro
        115                 120                 125

Gly Tyr Thr Tyr Ile Asn Pro Leu Asp Gly Thr Tyr Pro Pro Gly Thr
    130                 135                 140

Glu Gln Asn Pro Ala Asn Pro Asn Pro Ser Leu Glu Glu Ser Gln Pro
145                 150                 155                 160

Leu Asn Thr Phe Met Phe Gln Asn Asn Arg Phe Arg Asn Arg Gln Gly
                165                 170                 175

Ala Leu Thr Val Tyr Thr Gly Thr Val Thr Gln Gly Thr Asp Pro Val
            180                 185                 190

Lys Thr Tyr Tyr Gln Tyr Thr Pro Val Ser Ser Lys Ala Met Tyr Asp
        195                 200                 205

Ala Tyr Trp Asn Gly Lys Phe Arg Asp Cys Ala Phe His Ser Gly Phe
    210                 215                 220

Asn Glu Asp Pro Phe Val Cys Glu Tyr Gln Gly Gln Ser Ser Asp Leu
225                 230                 235                 240

Pro Gln Pro Pro Val Asn Ala Gly Gly Gly Ser Gly Gly Gly Ser Gly
                245                 250                 255

Gly Gly Ser Glu Gly Gly Gly Ser Glu Gly Gly Gly Ser Glu Gly Gly
            260                 265                 270

Gly Ser Glu Gly Gly Gly Ser Gly Gly Gly Ser Gly Ser Gly Asp Phe
        275                 280                 285

Asp Tyr Glu Lys Met Ala Asn Ala Asn Lys Gly Ala Met Thr Glu Asn
    290                 295                 300

Ala Asp Glu Asn Ala Leu Gln Ser Asp Ala Lys Gly Lys Leu Asp Ser
305                 310                 315                 320

Val Ala Thr Asp Tyr Gly Ala Ala Ile Asp Gly Phe Ile Gly Asp Val
                325                 330                 335

Ser Gly Leu Ala Asn Gly Asn Gly Ala Thr Gly Asp Phe Ala Gly Ser
            340                 345                 350

Asn Ser Gln Met Ala Gln Val Gly Asp Gly Asp Asn Ser Pro Leu Met
        355                 360                 365

Asn Asn Phe Arg Gln Tyr Leu Pro Ser Leu Pro Gln Ser Val Glu Cys
    370                 375                 380

Arg Pro Phe Val Phe Ser Ala Gly Lys Pro Tyr Glu Phe Ser Ile Asp
385                 390                 395                 400

Cys Asp Lys Ile Asn Leu Phe Arg Gly Val Phe Ala Phe Leu Leu Tyr
                405                 410                 415

Val Ala Thr Phe Met Tyr Val Phe Ser Thr Phe Ala Asn Ile Leu Arg
            420                 425                 430

Asn Lys Glu Ser
        435

<210>  11
<211>  1311
<212>  DNA
<213>  filamentous bacteriophage

<220>
<221>  misc_feature
<223>  n is any base


<220>
<221>  misc_feature
<222>  (55)..(90)
<223>  n is any base


<400>  11
gctgaaactg ttgaaagttg tttagcaaaa cccgatatcg accataagtc tggannntgc      60

nnnnnnnnnn nnnnnnnnnn nnnnnnnnnn tgcattgaag gtagactcca attgtctggt     120

acccatacag aaaattcatt tactaacgtc tggaaagacg acaaaacttt agatcgttac     180

gctaactatg agggttgtct gtggaatgct acaggcgttg tagtttgtac tggtgacgaa     240

actcagtgtt acggtacatg ggttcctatt gggcttgcta tccctgaaaa tgagggtggt     300

ggctctgagg gtggcggttc tgagggtggc ggttctgagg gtggcggtac taaacctcct     360

gagtacggtg atacacctat tccgggctat acttatatca accctctcga cggcacttat     420

ccgcctggta ctgagcaaaa ccccgctaat cctaatcctt ctcttgagga gtctcagcct     480

cttaatactt tcatgtttca gaataatagg ttccgaaata ggcagggggc attaactgtt     540

tatacgggca ctgttactca aggcactgac cccgttaaaa cttattacca gtacactcct     600

gtatcatcaa aagccatgta tgacgcttac tggaacggta aattcagaga ctgcgctttc     660

cattctggct ttaatgagga tccattcgtt tgtgaatatc aaggccaatc gtctgacctg     720

cctcaacctc ctgtcaatgc tggcggcggc tctggtggtg gttctggtgg cggctctgag     780

ggtggtggct ctgagggtgg cggttctgag ggtggcggct ctgagggagg cggttccggt     840

ggtggctctg gttccggtga ttttgattat gaaaagatgg caaacgctaa taagggggct     900

atgaccgaaa atgccgatga aaacgcgcta cagtctgacg ctaaaggcaa acttgattct     960

gtcgctactg attacggtgc tgctatcgat ggtttcattg gtgacgtttc cggccttgct    1020

aatggtaatg gtgctactgg tgattttgct ggctctaatt cccaaatggc tcaagtcggt    1080

gacggtgata attcaccttt aatgaataat ttccgtcaat atttaccttc cctccctcaa    1140

tcggttgaat gtcgcccttt tgtctttagc gctggtaaac catatgaatt ttctattgat    1200

tgtgacaaaa taaacttatt ccgtggtgtc tttgcgtttc ttttatatgt tgccaccttt    1260

atgtatgtat tttctacgtt tgctaacata ctgcgtaata aggagtctta a             1311


<210>  12
<211>  435
<212>  PRT
<213>  filamentous bacteriophage

<220>
<221>  MISC_FEATURE
<223>  X is any amino acid

<220>
<221>  MISC_FEATURE
<222>  (19)..(29)
<223>  X is any amino acid

<400>  12

Ala Glu Thr Val Glu Ser Cys Leu Ala Lys Pro Asp Ile Asp His Lys
1               5                   10                  15

Ser Gly Xaa Xaa Xaa Xaa Cys Xaa Xaa Xaa Xaa Xaa Xaa Cys Ile Glu
            20                  25                  30

Gly Arg Leu Gln Leu Ser Gly Thr His Thr Glu Asn Ser Phe Thr Asn
        35                  40                  45

Val Trp Lys Asp Asp Lys Thr Leu Asp Arg Tyr Ala Asn Tyr Glu Gly
    50                  55                  60

Cys Leu Trp Asn Ala Thr Gly Val Val Val Cys Thr Gly Asp Glu Thr
65                  70                  75                  80

Gln Cys Tyr Gly Thr Trp Val Pro Ile Gly Leu Ala Ile Pro Glu Asn
                85                  90                  95

Glu Gly Gly Gly Ser Glu Gly Gly Gly Ser Glu Gly Gly Gly Ser Glu
            100                 105                 110

Gly Gly Gly Thr Lys Pro Pro Glu Tyr Gly Asp Thr Pro Ile Pro Gly
        115                 120                 125

Tyr Thr Tyr Ile Asn Pro Leu Asp Gly Thr Tyr Pro Pro Gly Thr Glu
    130                 135                 140

Gln Asn Pro Ala Asn Pro Asn Pro Ser Leu Glu Glu Ser Gln Pro Leu
145                 150                 155                 160

Asn Thr Phe Met Phe Gln Asn Asn Arg Phe Arg Asn Arg Gln Gly Ala
                165                 170                 175

Leu Thr Val Tyr Thr Gly Thr Val Thr Gln Gly Thr Asp Pro Val Lys
            180                 185                 190

Thr Tyr Tyr Gln Tyr Thr Pro Val Ser Ser Lys Ala Met Tyr Asp Ala
        195                 200                 205

Tyr Trp Asn Gly Lys Phe Arg Asp Cys Ala Phe His Ser Gly Phe Asn
    210                 215                 220

Glu Asp Pro Phe Val Cys Glu Tyr Gln Gly Gln Ser Ser Asp Leu Pro
225                 230                 235                 240

Gln Pro Pro Val Asn Ala Gly Gly Gly Ser Gly Gly Gly Ser Gly Gly
                245                 250                 255

Gly Ser Glu Gly Gly Gly Ser Glu Gly Gly Gly Ser Glu Gly Gly Gly
            260                 265                 270

Ser Glu Gly Gly Gly Ser Gly Gly Gly Ser Gly Ser Gly Asp Phe Asp
        275                 280                 285

Tyr Glu Lys Met Ala Asn Ala Asn Lys Gly Ala Met Thr Glu Asn Ala
    290                 295                 300

Asp Glu Asn Ala Leu Gln Ser Asp Ala Lys Gly Lys Leu Asp Ser Val
305                 310                 315                 320

Ala Thr Asp Tyr Gly Ala Ala Ile Asp Gly Phe Ile Gly Asp Val Ser
                325                 330                 335

Gly Leu Ala Asn Gly Asn Gly Ala Thr Gly Asp Phe Ala Gly Ser Asn
            340                 345                 350

Ser Gln Met Ala Gln Val Gly Asp Gly Asp Asn Ser Pro Leu Met Asn
        355                 360                 365

Asn Phe Arg Gln Tyr Leu Pro Ser Leu Pro Gln Ser Val Glu Cys Arg
    370                 375                 380

Pro Phe Val Phe Ser Ala Gly Lys Pro Tyr Glu Phe Ser Ile Asp Cys
385                 390                 395                 400

Asp Lys Ile Asn Leu Phe Arg Gly Val Phe Ala Phe Leu Leu Tyr Val
                405                 410                 415

Ala Thr Phe Met Tyr Val Phe Ser Thr Phe Ala Asn Ile Leu Arg Asn
            420                 425                 430

Lys Glu Ser
        435

<210>  13
<211>  1308
<212>  DNA
<213>  filamentous bacteriophage

<220>
<221>  misc_feature
<223>  n is any base

<220>
<221>  misc_feature
<222>  (55)..(87)
<223>  n is any base

<400>  13
gctgaaactg ttgaaagttg tttagcaaaa cccgatatcg accataagtc tggannnnnn      60

nnnnnntgcn nnnnnnnnnn nnnnnnntgc attgaaggta gactccaatt gtctggtacc     120

catacagaaa attcatttac taacgtctgg aaagacgaca aaactttaga tcgttacgct     180

aactatgagg gttgtctgtg gaatgctaca ggcgttgtag tttgtactgg tgacgaaact     240

cagtgttacg gtacatgggt tcctattggg cttgctatcc ctgaaaatga gggtggtggc     300

tctgagggtg gcggttctga gggtggcggt tctgagggtg gcggtactaa acctcctgag     360

tacggtgata cacctattcc gggctatact tatatcaacc ctctcgacgg cacttatccg     420

cctggtactg agcaaaaccc cgctaatcct aatccttctc ttgaggagtc tcagcctctt     480

aatactttca tgtttcagaa taataggttc cgaaataggc agggggcatt aactgtttat     540

acgggcactg ttactcaagg cactgacccc gttaaaactt attaccagta cactcctgta     600

tcatcaaaag ccatgtatga cgcttactgg aacggtaaat tcagagactg cgctttccat     660

tctggcttta atgaggatcc attcgtttgt gaatatcaag gccaatcgtc tgacctgcct     720

caacctcctg tcaatgctgg cggcggctct ggtggtggtt ctggtggcgg ctctgagggt     780

ggtggctctg agggtggcgg ttctgagggt ggcggctctg agggaggcgg ttccggtggt     840

ggctctggtt ccggtgattt tgattatgaa aagatggcaa acgctaataa gggggctatg     900

accgaaaatg ccgatgaaaa cgcgctacag tctgacgcta aaggcaaact tgattctgtc     960

gctactgatt acggtgctgc tatcgatggt ttcattggtg acgtttccgg ccttgctaat    1020

ggtaatggtg ctactggtga ttttgctggc tctaattccc aaatggctca agtcggtgac    1080

ggtgataatt cacctttaat gaataatttc cgtcaatatt taccttccct ccctcaatcg    1140

gttgaatgtc gcccttttgt ctttagcgct ggtaaaccat atgaattttc tattgattgt    1200

gacaaaataa acttattccg tggtgtcttt gcgtttcttt tatatgttgc cacctttatg    1260

tatgtatttt ctacgtttgc taacatactg cgtaataagg agtcttaa                 1308

<210>  14
<211>  434
<212>  PRT
<213>  filamentous bacteriophage

<220>
<221>  MISC_FEATURE
<223>  X is any amino acid

<220>
<221>  MISC_FEATURE
<222>  (23)..(33)
<223>  X is any amino acid

<400>  14

Ala Glu Thr Val Glu Ser Cys Leu Ala Lys Pro Asp Ile Asp His Lys
1               5                   10                  15

Ser Gly Ile Glu Gly Arg Xaa Cys Xaa Xaa Xaa Xaa Cys Xaa Xaa Xaa
            20                  25                  30

Xaa Asp Gln Leu Ser Gly Thr His Thr Glu Asn Ser Phe Thr Asn Val
        35                  40                  45

Trp Lys Asp Asp Lys Thr Leu Asp Arg Tyr Ala Asn Tyr Glu Gly Cys
    50                  55                  60

Leu Trp Asn Ala Thr Gly Val Val Val Cys Thr Gly Asp Glu Thr Gln
65                  70                  75                  80

Cys Tyr Gly Thr Trp Val Pro Ile Gly Leu Ala Ile Pro Glu Asn Glu
                85                  90                  95

Gly Gly Gly Ser Glu Gly Gly Gly Ser Glu Gly Gly Gly Ser Glu Gly
            100                 105                 110

Gly Gly Thr Lys Pro Pro Glu Tyr Gly Asp Thr Pro Ile Pro Gly Tyr
        115                 120                 125

Thr Tyr Ile Asn Pro Leu Asp Gly Thr Tyr Pro Pro Gly Thr Glu Gln
    130                 135                 140

Asn Pro Ala Asn Pro Asn Pro Ser Leu Glu Glu Ser Gln Pro Leu Asn
145                 150                 155                 160

Thr Phe Met Phe Gln Asn Asn Arg Phe Arg Asn Arg Gln Gly Ala Leu
                165                 170                 175

Thr Val Tyr Thr Gly Thr Val Thr Gln Gly Thr Asp Pro Val Lys Thr
            180                 185                 190

Tyr Tyr Gln Tyr Thr Pro Val Ser Ser Lys Ala Met Tyr Asp Ala Tyr
        195                 200                 205

Trp Asn Gly Lys Phe Arg Asp Cys Ala Phe His Ser Gly Phe Asn Glu
    210                 215                 220

Asp Pro Phe Val Cys Glu Tyr Gln Gly Gln Ser Ser Asp Leu Pro Gln
225                 230                 235                 240

Pro Pro Val Asn Ala Gly Gly Gly Ser Gly Gly Gly Ser Gly Gly Gly
                245                 250                 255

Ser Glu Gly Gly Gly Ser Glu Gly Gly Gly Ser Glu Gly Gly Gly Ser
            260                 265                 270

Glu Gly Gly Gly Ser Gly Gly Gly Ser Gly Ser Gly Asp Phe Asp Tyr
        275                 280                 285

Glu Lys Met Ala Asn Ala Asn Lys Gly Ala Met Thr Glu Asn Ala Asp
    290                 295                 300

Glu Asn Ala Leu Gln Ser Asp Ala Lys Gly Lys Leu Asp Ser Val Ala
305                 310                 315                 320

Thr Asp Tyr Gly Ala Ala Ile Asp Gly Phe Ile Gly Asp Val Ser Gly
                325                 330                 335

Leu Ala Asn Gly Asn Gly Ala Thr Gly Asp Phe Ala Gly Ser Asn Ser
            340                 345                 350

Gln Met Ala Gln Val Gly Asp Gly Asp Asn Ser Pro Leu Met Asn Asn
        355                 360                 365

Phe Arg Gln Tyr Leu Pro Ser Leu Pro Gln Ser Val Glu Cys Arg Pro
    370                 375                 380

Phe Val Phe Ser Ala Gly Lys Pro Tyr Glu Phe Ser Ile Asp Cys Asp
385                 390                 395                 400

Lys Ile Asn Leu Phe Arg Gly Val Phe Ala Phe Leu Leu Tyr Val Ala
                405                 410                 415

Thr Phe Met Tyr Val Phe Ser Thr Phe Ala Asn Ile Leu Arg Asn Lys
            420                 425                 430

Glu Ser

<210>  15
<211>  1305
<212>  DNA
<213>  filamentous bacteriophage

<220>
<221>  misc_feature
<223>  n is any base

<220>
<221>  misc_feature
<222>  (67)..(99)
<223>  n is any base

<400>  15
gctgaaactg ttgaaagttg tttagcaaaa cccgatatcg accataagtc tggaattgaa      60

ggtagannnt gcnnnnnnnn nnnntgcnnn nnnnnnnnng accaattgtc tggtacccat     120

acagaaaatt catttactaa cgtctggaaa gacgacaaaa ctttagatcg ttacgctaac     180

tatgagggtt gtctgtggaa tgctacaggc gttgtagttt gtactggtga cgaaactcag     240

tgttacggta catgggttcc tattgggctt gctatccctg aaaatgaggg tggtggctct     300

gagggtggcg gttctgaggg tggcggttct gagggtggcg gtactaaacc tcctgagtac     360

ggtgatacac ctattccggg ctatacttat atcaaccctc tcgacggcac ttatccgcct 420

ggtactgagc aaaaccccgc taatcctaat ccttctcttg aggagtctca gcctcttaat 480

actttcatgt ttcagaataa taggttccga aataggcagg gggcattaac tgtttatacg 540

ggcactgtta ctcaaggcac tgaccccgtt aaaacttatt accagtacac tcctgtatca 600

tcaaaagcca tgtatgacgc ttactggaac ggtaaattca gagactgcgc tttccattct 660

ggctttaatg aggatccatt cgtttgtgaa tatcaaggcc aatcgtctga cctgcctcaa 720

cctcctgtca atgctggcgg cggctctggt ggtggttctg gtggcggctc tgagggtggt 780

ggctctgagg gtggcggttc tgagggtggc ggctctgagg gaggcggttc cggtggtggc 840

tctggttccg gtgattttga ttatgaaaag atggcaaacg ctaataaggg ggctatgacc 900

gaaaatgccg atgaaaacgc gctacagtct gacgctaaag gcaaacttga ttctgtcgct 960

actgattacg gtgctgctat cgatggtttc attggtgacg tttccggcct tgctaatggt 1020

aatggtgcta ctggtgattt tgctggctct aattcccaaa tggctcaagt cggtgacggt 1080

gataattcac ctttaatgaa taatttccgt caatatttac cttccctccc tcaatcggtt 1140

gaatgtcgcc cttttgtctt tagcgctggt aaaccatatg aattttctat tgattgtgac 1200

aaaataaact tattccgtgg tgtctttgcg tttcttttat atgttgccac ctttatgtat 1260

gtattttcta cgtttgctaa catactgcgt aataaggagt cttaa 1305

<210> 16
<211> 48
<212> DNA
<213> Artificial

<220>
<223> random mase

<400> 16
aaatgaattt tctgtatggg taccgatatc gggttttgct aaacaact 48

<210> 17
<211> 59
<212> DNA
<213> Artificial

<220>
<223> random base

<400> 17
ggatccgcag gatgcaagaa tttcttttgg aaaacgttca cctcctgtga tatcggtac 59

<210> 18
<211> 55
<212> DNA
<213> Artificial

<220>
<223> random base

<400> 18
cgatatcaca ggaggtgaac gttttccaaa agaaattctt gcatcctgcg gatcc 55

<210>  19
<211>  68
<212>  DNA
<213>  Artificial

<220>
<223>  random base

<400>  19
ggatccgctg gctgtaagaa tttcttttgg aaaacgttca catcatgcat cgagggaagg     60

accggtac                                                              68


<210>  20
<211>  24
<212>  DNA
<213>  Artificial

<220>
<223>  random base

<400>  20
cggtccttcc ctcgatgcat gatg                                            24


<210>  21
<211>  41
<212>  DNA
<213>  Artificial

<220>
<223>  random base

<400>  21
atcgaccata agtctggatc tagagaccaa ttgtctggta c                         41


<210>  22
<211>  37
<212>  DNA
<213>  Artificial

<220>
<223>  random base

<400>  22
cagacaattg gtctctagat ccagacttat ggtcgat                              37


<210>  23
<211>  51
<212>  DNA
<213>  Artificial

<220>
<223>  random base

<220>
<221>  misc_feature
<223>  n is any base


<220>
<221>  misc_feature
<222>  (12)..(23)

<223>  n is any base

<400>  23
gtaagaactt cnnnnnnnnn nnnttcacat cttgcattga aggtagattg t          51

<210>  24
<211>  15
<212>  DNA
<213>  Artificial

<220>
<223>  random base

<400>  24
gaagttctta cactt                                                  15

<210>  25
<211>  24
<212>  DNA
<213>  Artificial

<220>
<223>  random base

<400>  25
tctaccttca atgcaagatg tgaa                                        24

<210>  26
<211>  86
<212>  DNA
<213>  Artificial

<220>
<223>  random base

<220>
<221>  misc_feature
<222>  (23)..(64)
<223>  N is any amino acid

<400>  26
tgatatcgac cataagtctg gannnnnnnn nnnntgcnnn nnnnnnnnnt gcnnnnnnnn    60

nnnngaccaa ttgtctggta cccata                                      86

<210>  27
<211>  17
<212>  DNA
<213>  Artificial

<220>
<223>  random base

<400>  27
tgatatcgac cataagt                                                17

<210>  28
<211>  17
<212>  DNA
<213>  Artificial

<220>
<223>  random base

<400>  28
tatgggtacc agacaat                                                    17


<210>  29
<211>  88
<212>  DNA
<213>  Artificial

<220>
<223>  random base

<220>
<221>  misc_feature
<223>  n is any base


<220>
<221>  misc_feature
<222>  (22)..(57)
<223>  n is any base


<400>  29
gatatcgacc ataagtctgg annntgcnnn nnnnnnnnnn nnnnnnnnnn nnnnnnntgc      60

attgaaggta gactccaatt gtctggta                                        88


<210>  30
<211>  20
<212>  DNA
<213>  Artificial

<220>
<223>  random base

<400>  30
gatatcgacc ataagtctgg                                                 20


<210>  31
<211>  20
<212>  DNA
<213>  Artificial

<220>
<223>  random base

<400>  31
taccagacaa ttggagtcta                                                 20


<210>  32
<211>  85
<212>  DNA
<213>  Artificial

<220>
<223>  random base

<220>
<221>  misc_feature
<222>  (22)..(54)
<223>  n is any base

<400>  32
gatatcgacc ataagtctgg annnnnnnnn nnntgcnnnn nnnnnnnnnn nnnntgcatt      60

gaaggtagac tccaattgtc tggta                                            85


<210>  33
<211>  89
<212>  DNA
<213>  Artificial

<220>
<223>  random base

<220>
<221>  misc_feature
<222>  (35)..(67)
<223>  n is any base


<400>  33
tgatatcgac cataagtctg gaattgaagg tagannntgc nnnnnnnnnn nntgcnnnnn      60

nnnnnnngac caattgtctg gtacccata                                        89


<210>  34
<211>  431
<212>  PRT
<213>  filamentous bacteriophage

<220>
<221>  MISC_FEATURE
<222>  (21)..(24)
<223>  X is any amino acid


<400>  34

Ala Glu Thr Val Glu Ser Cys Leu Ala Lys Pro Asp Ile Asp His Lys
1               5                   10                  15


Cys Lys Asn Phe Xaa Xaa Xaa Xaa Phe Thr Ser Cys Ile Glu Gly Arg
            20                  25                  30


Leu Ser Gly Thr His Thr Glu Asn Ser Phe Thr Asn Val Trp Lys Asp
        35                  40                  45


Asp Lys Thr Leu Asp Arg Tyr Ala Asn Tyr Glu Gly Cys Leu Trp Asn
    50                  55                  60


Ala Thr Gly Val Val Val Cys Thr Gly Asp Glu Thr Gln Cys Tyr Gly
65                  70                  75                  80


Thr Trp Val Pro Ile Gly Leu Ala Ile Pro Glu Asn Glu Gly Gly Gly
                85                  90                  95


Ser Glu Gly Gly Gly Ser Glu Gly Gly Gly Ser Glu Gly Gly Gly Thr
            100                 105                 110

Lys Pro Pro Glu Tyr Gly Asp Thr Pro Ile Pro Gly Tyr Thr Tyr Ile
        115                 120                 125

Asn Pro Leu Asp Gly Thr Tyr Pro Pro Gly Thr Glu Gln Asn Pro Ala
    130                 135                 140

Asn Pro Asn Pro Ser Leu Glu Glu Ser Gln Pro Leu Asn Thr Phe Met
145                 150                 155                 160

Phe Gln Asn Asn Arg Phe Arg Asn Arg Gln Gly Ala Leu Thr Val Tyr
                165                 170                 175

Thr Gly Thr Val Thr Gln Gly Thr Asp Pro Val Lys Thr Tyr Tyr Gln
            180                 185                 190

Tyr Thr Pro Val Ser Ser Lys Ala Met Tyr Asp Ala Tyr Trp Asn Gly
        195                 200                 205

Lys Phe Arg Asp Cys Ala Phe His Ser Gly Phe Asn Glu Asp Pro Phe
    210                 215                 220

Val Cys Glu Tyr Gln Gly Gln Ser Ser Asp Leu Pro Gln Pro Pro Val
225                 230                 235                 240

Asn Ala Gly Gly Gly Ser Gly Gly Gly Ser Gly Gly Gly Ser Glu Gly
                245                 250                 255

Gly Gly Ser Glu Gly Gly Gly Ser Glu Gly Gly Gly Ser Glu Gly Gly
            260                 265                 270

Gly Ser Gly Gly Gly Ser Gly Ser Gly Asp Phe Asp Tyr Glu Lys Met
        275                 280                 285

Ala Asn Ala Asn Lys Gly Ala Met Thr Glu Asn Ala Asp Glu Asn Ala
    290                 295                 300

Leu Gln Ser Asp Ala Lys Gly Lys Leu Asp Ser Val Ala Thr Asp Tyr
305                 310                 315                 320

Gly Ala Ala Ile Asp Gly Phe Ile Gly Asp Val Ser Gly Leu Ala Asn
                325                 330                 335

Gly Asn Gly Ala Thr Gly Asp Phe Ala Gly Ser Asn Ser Gln Met Ala
            340                 345                 350

Gln Val Gly Asp Gly Asp Asn Ser Pro Leu Met Asn Asn Phe Arg Gln
        355                 360                 365

Tyr Leu Pro Ser Leu Pro Gln Ser Val Glu Cys Arg Pro Phe Val Phe
    370                 375                 380

Ser Ala Gly Lys Pro Tyr Glu Phe Ser Ile Asp Cys Asp Lys Ile Asn
385                 390                 395                 400

Leu Phe Arg Gly Val Phe Ala Phe Leu Leu Tyr Val Ala Thr Phe Met
                405                 410                 415

Tyr Val Phe Ser Thr Phe Ala Asn Ile Leu Arg Asn Lys Glu Ser
            420                 425                 430

<210>  35
<211>  433
<212>  PRT
<213>  filamentous bacteriophage

<220>
<221>  MISC_FEATURE
<222>  (19)..(32)
<223>  X is any amino acid

<400>  35

Ala Glu Thr Val Glu Ser Cys Leu Ala Lys Pro Asp Ile Asp His Lys
1               5                   10                  15

Ser Gly Xaa Xaa Xaa Xaa Cys Xaa Xaa Xaa Xaa Cys Xaa Xaa Xaa Xaa
            20                  25                  30

Asp Gln Leu Ser Gly Thr His Thr Glu Asn Ser Phe Thr Asn Val Trp
        35                  40                  45

Lys Asp Asp Lys Thr Leu Asp Arg Tyr Ala Asn Tyr Glu Gly Cys Leu
    50                  55                  60

Trp Asn Ala Thr Gly Val Val Val Cys Thr Gly Asp Glu Thr Gln Cys
65                  70                  75                  80

Tyr Gly Thr Trp Val Pro Ile Gly Leu Ala Ile Pro Glu Asn Glu Gly
                85                  90                  95

Gly Gly Ser Glu Gly Gly Gly Ser Glu Gly Gly Gly Ser Glu Gly Gly
            100                 105                 110

Gly Thr Lys Pro Pro Glu Tyr Gly Asp Thr Pro Ile Pro Gly Tyr Thr
        115                 120                 125

Tyr Ile Asn Pro Leu Asp Gly Thr Tyr Pro Pro Gly Thr Glu Gln Asn
    130                 135                 140

Pro Ala Asn Pro Asn Pro Ser Leu Glu Glu Ser Gln Pro Leu Asn Thr
145                 150                 155                 160

Phe Met Phe Gln Asn Asn Arg Phe Arg Asn Arg Gln Gly Ala Leu Thr
                165                 170                 175

Val Tyr Thr Gly Thr Val Thr Gln Gly Thr Asp Pro Val Lys Thr Tyr
            180                 185                 190

Tyr Gln Tyr Thr Pro Val Ser Ser Lys Ala Met Tyr Asp Ala Tyr Trp
        195                 200                 205

Asn Gly Lys Phe Arg Asp Cys Ala Phe His Ser Gly Phe Asn Glu Asp
    210                 215                 220

Pro Phe Val Cys Glu Tyr Gln Gly Gln Ser Ser Asp Leu Pro Gln Pro
225                 230                 235                 240

Pro Val Asn Ala Gly Gly Gly Ser Gly Gly Gly Ser Gly Gly Gly Ser
                245                 250                 255

Glu Gly Gly Gly Ser Glu Gly Gly Gly Ser Glu Gly Gly Gly Ser Glu
            260                 265                 270

Gly Gly Gly Ser Gly Gly Gly Ser Gly Ser Gly Asp Phe Asp Tyr Glu
        275                 280                 285

Lys Met Ala Asn Ala Asn Lys Gly Ala Met Thr Glu Asn Ala Asp Glu
    290                 295                 300

Asn Ala Leu Gln Ser Asp Ala Lys Gly Lys Leu Asp Ser Val Ala Thr
305                 310                 315                 320

Asp Tyr Gly Ala Ala Ile Asp Gly Phe Ile Gly Asp Val Ser Gly Leu
                325                 330                 335

Ala Asn Gly Asn Gly Ala Thr Gly Asp Phe Ala Gly Ser Asn Ser Gln
            340                 345                 350

Met Ala Gln Val Gly Asp Gly Asp Asn Ser Pro Leu Met Asn Asn Phe
        355                 360                 365

Arg Gln Tyr Leu Pro Ser Leu Pro Gln Ser Val Glu Cys Arg Pro Phe
    370                 375                 380

Val Phe Ser Ala Gly Lys Pro Tyr Glu Phe Ser Ile Asp Cys Asp Lys
385                 390                 395                 400

Ile Asn Leu Phe Arg Gly Val Phe Ala Phe Leu Leu Tyr Val Ala Thr
                405                 410                 415

Phe Met Tyr Val Phe Ser Thr Phe Ala Asn Ile Leu Arg Asn Lys Glu
            420                 425                 430

Ser

<210>  36
<211>  436
<212>  PRT
<213>  filamentous bacteriophage

<220>
<221>  MISC_FEATURE
<222>  (19)..(30)
<223>  X is any amino acid


<400>  36

Ala Glu Thr Val Glu Ser Cys Leu Ala Lys Pro Asp Ile Asp His Lys
1               5                   10                  15

Ser Gly Xaa Cys Xaa Xaa Xaa Xaa Xaa Xaa Xaa Xaa Xaa Xaa Cys Ile
            20                  25                  30

Glu Gly Arg Leu Gln Leu Ser Gly Thr His Thr Glu Asn Ser Phe Thr
        35                  40                  45

Asn Val Trp Lys Asp Asp Lys Thr Leu Asp Arg Tyr Ala Asn Tyr Glu
    50                  55                  60

Gly Cys Leu Trp Asn Ala Thr Gly Val Val Val Cys Thr Gly Asp Glu
65                  70                  75                  80

Thr Gln Cys Tyr Gly Thr Trp Val Pro Ile Gly Leu Ala Ile Pro Glu
                85                  90                  95

Asn Glu Gly Gly Gly Ser Glu Gly Gly Gly Ser Glu Gly Gly Gly Ser
            100                 105                 110

Glu Gly Gly Gly Thr Lys Pro Pro Glu Tyr Gly Asp Thr Pro Ile Pro
        115                 120                 125

Gly Tyr Thr Tyr Ile Asn Pro Leu Asp Gly Thr Tyr Pro Pro Gly Thr
    130                 135                 140

Glu Gln Asn Pro Ala Asn Pro Asn Pro Ser Leu Glu Glu Ser Gln Pro
145                 150                 155                 160

Leu Asn Thr Phe Met Phe Gln Asn Asn Arg Phe Arg Asn Arg Gln Gly
                165                 170                 175

Ala Leu Thr Val Tyr Thr Gly Thr Val Thr Gln Gly Thr Asp Pro Val
            180                 185                 190

Lys Thr Tyr Tyr Gln Tyr Thr Pro Val Ser Ser Lys Ala Met Tyr Asp
        195                 200                 205

Ala Tyr Trp Asn Gly Lys Phe Arg Asp Cys Ala Phe His Ser Gly Phe

210                 215                 220

Asn Glu Asp Pro Phe Val Cys Glu Tyr Gln Gly Gln Ser Ser Asp Leu
225                 230                 235                 240

Pro Gln Pro Pro Val Asn Ala Gly Gly Gly Ser Gly Gly Gly Ser Gly
                245                 250                 255

Gly Gly Ser Glu Gly Gly Gly Ser Glu Gly Gly Gly Ser Glu Gly Gly
            260                 265                 270

Gly Ser Glu Gly Gly Gly Ser Gly Gly Gly Ser Gly Ser Gly Asp Phe
        275                 280                 285

Asp Tyr Glu Lys Met Ala Asn Ala Asn Lys Gly Ala Met Thr Glu Asn
    290                 295                 300

Ala Asp Glu Asn Ala Leu Gln Ser Asp Ala Lys Gly Lys Leu Asp Ser
305                 310                 315                 320

Val Ala Thr Asp Tyr Gly Ala Ala Ile Asp Gly Phe Ile Gly Asp Val
                325                 330                 335

Ser Gly Leu Ala Asn Gly Asn Gly Ala Thr Gly Asp Phe Ala Gly Ser
            340                 345                 350

Asn Ser Gln Met Ala Gln Val Gly Asp Gly Asp Asn Ser Pro Leu Met
        355                 360                 365

Asn Asn Phe Arg Gln Tyr Leu Pro Ser Leu Pro Gln Ser Val Glu Cys
    370                 375                 380

Arg Pro Phe Val Phe Ser Ala Gly Lys Pro Tyr Glu Phe Ser Ile Asp
385                 390                 395                 400

Cys Asp Lys Ile Asn Leu Phe Arg Gly Val Phe Ala Phe Leu Leu Tyr
                405                 410                 415

Val Ala Thr Phe Met Tyr Val Phe Ser Thr Phe Ala Asn Ile Leu Arg
            420                 425                 430

Asn Lys Glu Ser
        435

<210>  37
<211>  435
<212>  PRT
<213>  filamentous bacteriophage

<220>
<221>  MISC_FEATURE
<222>  (19)..(29)
<223>  X is any amino acid

<400> 37

Ala Glu Thr Val Glu Ser Cys Leu Ala Lys Pro Asp Ile Asp His Lys
1               5                   10                  15

Ser Gly Xaa Xaa Xaa Xaa Cys Xaa Xaa Xaa Xaa Xaa Xaa Cys Ile Glu
            20                  25                  30

Gly Arg Leu Gln Leu Ser Gly Thr His Thr Glu Asn Ser Phe Thr Asn
        35                  40                  45

Val Trp Lys Asp Asp Lys Thr Leu Asp Arg Tyr Ala Asn Tyr Glu Gly
    50                  55                  60

Cys Leu Trp Asn Ala Thr Gly Val Val Val Cys Thr Gly Asp Glu Thr
65                  70                  75                  80

Gln Cys Tyr Gly Thr Trp Val Pro Ile Gly Leu Ala Ile Pro Glu Asn
                85                  90                  95

Glu Gly Gly Gly Ser Glu Gly Gly Gly Ser Glu Gly Gly Gly Ser Glu
            100                 105                 110

Gly Gly Gly Thr Lys Pro Pro Glu Tyr Gly Asp Thr Pro Ile Pro Gly
        115                 120                 125

Tyr Thr Tyr Ile Asn Pro Leu Asp Gly Thr Tyr Pro Pro Gly Thr Glu
    130                 135                 140

Gln Asn Pro Ala Asn Pro Asn Pro Ser Leu Glu Glu Ser Gln Pro Leu
145                 150                 155                 160

Asn Thr Phe Met Phe Gln Asn Asn Arg Phe Arg Asn Arg Gln Gly Ala
                165                 170                 175

Leu Thr Val Tyr Thr Gly Thr Val Thr Gln Gly Thr Asp Pro Val Lys
            180                 185                 190

Thr Tyr Tyr Gln Tyr Thr Pro Val Ser Ser Lys Ala Met Tyr Asp Ala
        195                 200                 205

Tyr Trp Asn Gly Lys Phe Arg Asp Cys Ala Phe His Ser Gly Phe Asn
    210                 215                 220

Glu Asp Pro Phe Val Cys Glu Tyr Gln Gly Gln Ser Ser Asp Leu Pro
225                 230                 235                 240

Gln Pro Pro Val Asn Ala Gly Gly Gly Ser Gly Gly Gly Ser Gly Gly
                245                 250                 255

Gly Ser Glu Gly Gly Gly Ser Glu Gly Gly Gly Ser Glu Gly Gly Gly
            260                 265                 270

Ser Glu Gly Gly Gly Ser Gly Gly Gly Ser Gly Ser Gly Asp Phe Asp
        275                 280                 285

Tyr Glu Lys Met Ala Asn Ala Asn Lys Gly Ala Met Thr Glu Asn Ala
    290                 295                 300

Asp Glu Asn Ala Leu Gln Ser Asp Ala Lys Gly Lys Leu Asp Ser Val
305                 310                 315                 320

Ala Thr Asp Tyr Gly Ala Ala Ile Asp Gly Phe Ile Gly Asp Val Ser
                325                 330                 335

Gly Leu Ala Asn Gly Asn Gly Ala Thr Gly Asp Phe Ala Gly Ser Asn
            340                 345                 350

Ser Gln Met Ala Gln Val Gly Asp Gly Asp Asn Ser Pro Leu Met Asn
        355                 360                 365

Asn Phe Arg Gln Tyr Leu Pro Ser Leu Pro Gln Ser Val Glu Cys Arg
    370                 375                 380

Pro Phe Val Phe Ser Ala Gly Lys Pro Tyr Glu Phe Ser Ile Asp Cys
385                 390                 395                 400

Asp Lys Ile Asn Leu Phe Arg Gly Val Phe Ala Phe Leu Leu Tyr Val
                405                 410                 415

Ala Thr Phe Met Tyr Val Phe Ser Thr Phe Ala Asn Ile Leu Arg Asn
            420                 425                 430

Lys Glu Ser
        435

<210>  38
<211>  434
<212>  PRT
<213>  filamentous bacteriophage

<220>
<221>  MISC_FEATURE
<222>  (23)..(33)
<223>  X is any amino acid

<400>  38

Ala Glu Thr Val Glu Ser Cys Leu Ala Lys Pro Asp Ile Asp His Lys
1               5                   10                  15

Ser Gly Ile Glu Gly Arg Xaa Cys Xaa Xaa Xaa Xaa Cys Xaa Xaa Xaa
            20                  25                  30

Xaa Asp Gln Leu Ser Gly Thr His Thr Glu Asn Ser Phe Thr Asn Val
            35                  40                  45

Trp Lys Asp Asp Lys Thr Leu Asp Arg Tyr Ala Asn Tyr Glu Gly Cys
    50                  55                  60

Leu Trp Asn Ala Thr Gly Val Val Val Cys Thr Gly Asp Glu Thr Gln
65                  70                  75                  80

Cys Tyr Gly Thr Trp Val Pro Ile Gly Leu Ala Ile Pro Glu Asn Glu
                85                  90                  95

Gly Gly Gly Ser Glu Gly Gly Gly Ser Glu Gly Gly Gly Ser Glu Gly
            100                 105                 110

Gly Gly Thr Lys Pro Pro Glu Tyr Gly Asp Thr Pro Ile Pro Gly Tyr
        115                 120                 125

Thr Tyr Ile Asn Pro Leu Asp Gly Thr Tyr Pro Pro Gly Thr Glu Gln
    130                 135                 140

Asn Pro Ala Asn Pro Asn Pro Ser Leu Glu Glu Ser Gln Pro Leu Asn
145                 150                 155                 160

Thr Phe Met Phe Gln Asn Asn Arg Phe Arg Asn Arg Gln Gly Ala Leu
                165                 170                 175

Thr Val Tyr Thr Gly Thr Val Thr Gln Gly Thr Asp Pro Val Lys Thr
            180                 185                 190

Tyr Tyr Gln Tyr Thr Pro Val Ser Ser Lys Ala Met Tyr Asp Ala Tyr
        195                 200                 205

Trp Asn Gly Lys Phe Arg Asp Cys Ala Phe His Ser Gly Phe Asn Glu
    210                 215                 220

Asp Pro Phe Val Cys Glu Tyr Gln Gly Gln Ser Ser Asp Leu Pro Gln
225                 230                 235                 240

Pro Pro Val Asn Ala Gly Gly Gly Ser Gly Gly Gly Ser Gly Gly Gly
                245                 250                 255

Ser Glu Gly Gly Gly Ser Glu Gly Gly Gly Ser Glu Gly Gly Gly Ser
            260                 265                 270

Glu Gly Gly Gly Ser Gly Gly Gly Ser Gly Ser Gly Asp Phe Asp Tyr
        275                 280                 285

Glu Lys Met Ala Asn Ala Asn Lys Gly Ala Met Thr Glu Asn Ala Asp
    290                 295                 300

Glu Asn Ala Leu Gln Ser Asp Ala Lys Gly Lys Leu Asp Ser Val Ala
305                 310                 315                 320

Thr Asp Tyr Gly Ala Ala Ile Asp Gly Phe Ile Gly Asp Val Ser Gly
                325                 330                 335

Leu Ala Asn Gly Asn Gly Ala Thr Gly Asp Phe Ala Gly Ser Asn Ser
            340                 345                 350

Gln Met Ala Gln Val Gly Asp Gly Asp Asn Ser Pro Leu Met Asn Asn
        355                 360                 365

Phe Arg Gln Tyr Leu Pro Ser Leu Pro Gln Ser Val Glu Cys Arg Pro
    370                 375                 380

Phe Val Phe Ser Ala Gly Lys Pro Tyr Glu Phe Ser Ile Asp Cys Asp
385                 390                 395                 400

Lys Ile Asn Leu Phe Arg Gly Val Phe Ala Phe Leu Leu Tyr Val Ala
                405                 410                 415

Thr Phe Met Tyr Val Phe Ser Thr Phe Ala Asn Ile Leu Arg Asn Lys
            420                 425                 430

Glu Ser

## Description

### TECHNICAL FIELD

The present invention relates to a novel phage vector and a phage display method using the vector.

### BACKGROUND

When peptides are fused to the N-terminus of the pIII protein of a M13 phage, the peptides are displayed on M13 phage particles. This is known as the "phage display technique." By having the peptides that are fused be random peptides, a peptide library can be constructed, which provides a source for screening ligands (Scott, J.K. and Smith, G.P. (1990): "Searching for peptide ligands with an epitope library," Science 249, 386-90).

The phage display technique can in theory provide an enormous number of small peptides, and the peptide libraries thereby obtained generate diversity in the primary structure of the peptides. Many developments are underway in M13 phage display technology. Indeed, a variety of vectors for peptide display have already been developed (Smith, G.P. and Petrenko, V.A. (1997): "Phage Display," Chem. Rev. 97, 391-410).

In phage display techniques using M13 phages, fusion between pIII and a peptide is localized at the N-terminus or its immediate vicinity on a mature pIII or defective pIII. Because fusion proteins of pIII and a peptide are subject to influences by the peptide in the secretion process, it is thought to be impossible to display all peptides on a phage. As a result, a biological bias in peptide diversity is generated (Wilson, D.R. and Finlay, B.B. (1998): "Phage display: applications, innovations, and issues in phage and host biology," Can. J. Microbiol. 44, 313-29).

Hence, limitations arising from the amino acid sequence of the random peptide at or near the N-terminus are sometimes imposed on the periplasmic secretion of a random fusion peptide (Li, P., Beckwith, J. and Inouye, H. (1988): "Alternation of the amino terminus of the mature sequence of a periplasmic protein can severely affect protein export in Escherichia coli," Proc. Natl. Acad. Sci. USA 85, 7685-9; and Peters, E.A., Schatz, P.J., Johnson, S.S. and Dower, W.J. (1994): "Membrane insertion defects caused by positive charges in the early mature region of protein pIII of filamentous phage fd can be corrected by prlA suppressors," J. Bacteriol. 176, 4296-305).

This bias becomes smaller the further the random peptide insertion site is from the N-terminus (Summers, R.G. and Knowles, J.R. (1989): "Illicit secretion of a cytoplasmic protein into the periplasm of Escherichia coli requires a signal peptide plus a portion of the cognate secreted protein. Demarcation of the critical region of the mature protein," J. Biol. Chem. 264, 20074-81). Therefore, when fusing pIII and a peptide, at least with regard to secretion of the fusion protein, fusion of the peptide further inward than the N-terminal fusion of mature pIII is thought to be superior for secretion of the fusion protein. In this way, reducing biological constraints plays an important role in peptide diversity within phage display technology.

Peptides exist widely in the biological world as ligands capable of binding to proteins, and are employed in, for example, drug products and reagents. For a peptide to function as a ligand, it is necessary to form not only the primary structure of the peptide, but also the peptide conformation. In a peptide, the primary structure refers to the amino acid sequence, and the conformation refers to the geometrical structure of the overall molecule when a peptide chain forms a folded structure due to intramolecular interactions between the amino acids present on the molecule. When a peptide has been rendered into a fusion protein, the peptide conformation depends strongly not only on the primary structure of the peptide, but also on the conformation of the overall fusion protein. This dependency is determined by the site of peptide fusion in the fusion protein.

The diversity of antibodies is an appropriate illustration of this. The antigen-binding site in an antibody is a peptide called the complementarity-determining region (CDR). This peptide exists at the interior of a Fab protein, and the peptide conformation is formed according to both the primary structure of the peptide and the Fab protein scaffold. Accordingly, antibodies, through the diversity of the CDR peptide conformation and primary structure, generate the ability to bind to all target proteins. Even in a fusion protein, the conformation of a peptide fused at the N-terminus or C-terminus is relatively little affected by the conformation of the overall fusion protein. In this way, the conformation of a peptide at the interior of a fusion protein is strongly influenced by the conformation of the overall fusion protein. In other words, the conformation of a peptide at the interior of a fusion protein incurs large conformational constraints from the fusion protein. It is from such constraints that distinctive conformations are created in peptides.

Disulfide linkages form between cysteine residues at specific sites, causing the peptide to cyclize and assume a particular conformation. The formation of disulfide linkages at specific sites exerts a large influence on the conformation of the peptide between the disulfide linkages.

The conformation of a peptide is thus determined primarily by three factors: the primary structure of the peptide, conformational constraints from the over fusion protein, and the disulfide linkages.

When a random peptide is produced by an M13 phage, the primary structure of the peptide and the formation of disulfide linkages are determined by inserts (insertion peptides), and the conformational constraints from the fusion protein are determined by the M13 phage vector. Currently developed M13 phage vectors all carry out N-terminal or C-terminal fusion; almost none are subject to conformational constraints from the fusion protein. Hence, the peptide conformation in currently developed M13 phage displays is strongly dependent on the primary structure of the peptide. In M13 phage display, the development of M13 phage vectors which incur conformational constraints from the overall fusion protein, i.e., the development of M13 phage vectors in which the peptide can fuse at the interior of pIII, will create novel peptide conformations in a random peptide libraries.

There exist two basic approaches for displaying random peptides in the phage display technique. One approach involves fusing a cyclized random peptide to the N-terminus of pIII; this is used to construct almost all random peptide libraries (Smith, G.P. and Petrenko, V.A. (1997): "Phage display," Chem. Rev. 97, 391-410). In the other approach, a specific protein is fused to the N-terminus of pIII and a portion of the protein structure is replaced with a random peptide. The latter approach is exemplified by a method carried out using tendamistat as a protein scaffold (McConnell, S.J. and Hoess, R.H. (1995): "Tendamistat as a scaffold for conformationally constrained phage peptide libraries," J. Mol. Biol. 250, 460-70). In the tendamistat fusion method, the turn structure in tendamistat is replaced with a random peptide. Phage vectors capable of fusing a peptide at the interior of pIII in this way have not yet been reported in the literature.

pIII is a gene-3-protein (g3p) of filamentous bacteriophage which takes part in phage infection (Armstrong, J., Perham, R.N. and Walker, J.E. (1981): "Domain structure of bacteriophage fd adsorption protein," FEBS Lett. 135, 167-72). It has been shown that there are five pIII molecules per phage, each molecule being composed of 406 amino acids and having a crystalline structure with N1, N2 and C domains (Lubkowski, J., Hennecke, F., Pluckthun, A. and Wlodawer, A. (1999): "Filamentous phage infection: Crystal structure of g3p in complex with its coreceptor, the C-terminal domain of TolA," Structure 7, 711-22; Holliger, P., Riechmann, L. and Williams, R.L. (1999): "Crystal structure of the two N-terminal domains of g3p from filamentous phage fd at 1.9A: Evidence for conformational lability," J. Mol. Biol. 288, 649-57; and Lubkowski, J., Hennecke, F., Pluckthun, A and Wlodawer, A (1998): "The structural basis of phage display elucidated by the crystal structure of the N-terminal domains of g3p," Nat. Struct. Biol. 5, 140-147). The N1 and N2 domains respectively have, in phage infection, an E. coli cell wall penetrating action and an E. coli attaching action (Armstrong, J., Perham, R.N. and Walker, J.E. (1981): "Domain structure of bacteriophage fd adsorption protein," FEBS Lett. 135, 167-72; and Stengele, I., Bross, P., Garces, X. Giray, J. and Rasched, I. (1990): "Dissection of functional domains in phage fd adsorption protein. Discrimination between attachment and penetration sites," J. Mol. Biol. 212, 143-9). The C domain takes part in phage particle formation via interactions between the phage and the g6p molecule (Armstrong, J., Perham, R.N. and Walker, J.E. (1981): "Domain structure of bacteriophage fd adsorption protein," FEBS Lett. 135, 167-72; Kremser, A. and Rasched, I. (1994): "The adsorption protein of filamentous phage fd: Assignment of its disulfide bridges and identification of the domain incorporated in the coat," Biochemistry 33, 13954-8; Nelson, F.K., Friedman, S.M. and Smith, G.P. (1981): "Filamentous phage DNA cloning vectors: A noninfective mutant with a nonpolar deletion in gene III," Virology 108, 338-50). Because pIII carries out in this way the essential function of phage infection, producing mutants while retaining this function is thought to be extremely difficult. In particular, with the dissociation of the C7-C36 disulfide linkage that exists in the N1 domain, the phage loses its infectivity; i.e., a change in the conformation of the N1 domain leads to a loss of phage infectivity (Kather, I., Bippes, C.A. and Schmid, F.X. (2005): "A stable disulfide-free gene-3-protein of phage fd generated by in vitro evolution," J. Mol. Biol. 354, 666-78).

### DISCLOSURE OF THE INVENTION

An object of the present invention is to obtain, in phage display, a phase display vector capable of fusing random peptides at the interior of pIII so as to construct novel random peptide libraries. A further object of the invention is to provide a phage display method which uses such a vector. A still further object of the invention is to provide a method for obtaining peptide libraries by such a phage display method.

The inventor has discovered filamentous bacteriophage vectors which allow a peptide to be inserted at the interior of the N-terminal domain of a pIII protein, and have created novel displays of peptides. Insertion mutagenesis using single-stranded DNA from the M13K07 phage was employed to search for sites at the interior of the N-terminal domain of pIII where peptide insertion is possible. As a result, mutant bacteriophages having a peptide inserted at one such site, located between the proline residue at position 11 and the histidine residue at position 12 on mature pIII, were obtained. It was possible to insert a peptide having up to 30 amino acid residues at this insertion site in the mutant phages without a loss in the phage infection and growth functions.

The inventor also inserted synthetic DNA coding for somatostatin-14 into the mutant phages, and investigated the display of somatostatin-14 on the phages. The somatostatin-14 display on the phages was identified by ELISA using an anti-somatostatin antibody. The fusion protein of pIII and somatostatin-14 was identified by Western blotting using an anti-somatostatin antibody.

In addition, random peptide libraries were created using the somatostatin-14 displayed on the phages as the peptide scaffold. The phage display libraries were constructed by replacing the FWKT residues that are the center of activity on somatostatin-14 with a random tetrapeptide. In addition, it was possible to introduce random peptides of general formula C(X)nC, and the subsequently described M13yt42 vector was able to create peptide libraries having diverse disulfide structures with peptide sizes of up to 30 amino acid residues.

Also, concerning the peptide libraries obtained, the frequencies of amino acid residues within random sequences in 171 appropriately selected clones were analyzed in order to investigate the diversity of the amino acid sequences. The results showed that there was a high positive correlation between the amino acid frequencies predicted from codons which encode the random sequences and the measured amino acid frequencies in random sequences from clones. This indicates that the production of libraries using the M13yt42 vector is not readily subject to a biological bias, which is a major advantage in the expression of random peptides. N-terminal fusion-type phage libraries up until now were characterized by a high frequency of proline residues in cyclic random sequences due to disulfide linkages, whereas the amino acid frequencies within the present libraries are characterized by the frequent appearance of a single glycine residue. Because the M13yt42 vector is able in this way to display a peptide at the interior of the N-terminal domain of pIII, along with the above-indicated advantage in terms of peptide expression, it is possible to display random peptide sequences which, unlike N-terminal fusion-type M13 libraries up until now, are rich in glycine and to some degree conformationally constrained.

Accordingly, the present invention relates to a mutant pIII protein having an amino acid residue inserted between a proline residue at position 11 and a histidine residue at position 12 of a filamentous bacteriophage pIII protein of SEQ ID NO: 1.
The invention also relates to the foregoing mutant pIII protein, wherein from 1 to 50 amino acid residues are inserted.
The invention also relates to the foregoing mutant pIII protein, wherein from 4 to 30 amino acid residues are inserted.
The invention also relates to a polypeptide of SEQ ID NO: 34 (wherein each X in the sequence table is independently any amino acid residue).
The invention also relates to a polypeptide of SEQ ID NO: 35 (wherein each X in the sequence table is independently any amino acid residue).
The invention also relates to a polypeptide of SEQ ID NO: 36 (wherein each X in the sequence table is independently any amino acid residue).
The invention also relates to a polypeptide of SEQ ID NO: 37 (wherein each X in the sequence table is independently any amino acid residue).
The invention also relates to a polypeptide of SEQ ID NO: 38 (wherein each X in the sequence table is independently any amino acid residue).
The invention also relates to the foregoing polypeptides wherein each X is independently an amino acid residue selected from the group consisting of A, C, D, E, F, G, H, I, K, L, M, N, P, Q, R, S, T, V, W and Y.
The invention also relates to a polypeptide of SEQ ID NO: 2.
The invention also relates to a polypeptide of SEQ ID NO: 3.
The invention also relates to a polypeptide of SEQ ID NO: 4.
The invention also relates to a polypeptide of SEQ ID NO: 5.
The invention also relates to a polynucleotide which codes for the above mutant pIII protein.
The invention also relates to a polynucleotide which codes for the polypeptide of any one of claims 4 to 13.
The invention also relates to a vector containing the above polynucleotide.
The invention also relates to the above vector which is a filamentous bacteriophage.
The invention also relates to a host cell containing the above vector.
The invention also relates to the above host cell which is Escherichia coli.
The invention also relates to a polypeptide library containing two or more polypeptides of SEQ ID NO: 34 (wherein each X in the sequence table is independently any amino acid residue).
The invention also relates to a polypeptide library containing two or more polypeptides of SEQ ID NO: 35 (wherein each X in the sequence table is independently any amino acid residue).
The invention also relates to a polypeptide library containing two or more polypeptides of SEQ ID NO: 36 (wherein each X in the sequence table is independently any amino acid residue).
The invention also relates to a polypeptide library containing two or more polypeptides of SEQ ID NO: 37 (wherein each X in the sequence table is independently any amino acid residue).
The invention also relates to a polypeptide library containing two or more polypeptides of SEQ ID NO: 38 (wherein each X in the sequence table is independently any amino acid residue).
The invention also relates to the foregoing polypeptide libraries wherein each X is independently an amino acid residue selected from the group consisting of A, C, D, E, F, G, H, I, K, L, M, N, P, Q, R, S, T, V, W and Y.
The invention also relates to a filamentous bacteriophage containing the above polynucleotide.
The invention also relates to a filamentous bacteriophage which displays the above mutant pIII protein.
The invention also relates to a filamentous bacteriophage which displays the above polypeptide.
The invention also relates to the above filamentous bacteriophage, which filamentous bacteriophage is of at least one type selected from the group consisting of fl phages, fd phages and M 13 phages.
The invention also relates to the above filamentous bacteriophage, which filamentous bacteriophage is an M13 phage.
The invention also relates to a filamentous bacteriophage library which displays two or more of the above-mentioned mutant pIII proteins.
The invention also relates to a filamentous bacteriophage library containing two or more polypeptides of SEQ ID NO: 34 (wherein each X in the sequence table is independently any amino acid residue).
The invention also relates to a filamentous bacteriophage library containing two or more polypeptides of SEQ ID NO: 35 (wherein each X in the sequence table is independently any amino acid residue).
The invention also relates to a filamentous bacteriophage library containing two or more polypeptides of SEQ ID NO: 36 (wherein each X in the sequence table is independently any amino acid residue).
The invention also relates to a filamentous bacteriophage library containing two or more polypeptides of SEQ ID NO: 37 (wherein each X in the sequence table is independently any amino acid residue).
The invention also relates to a filamentous bacteriophage library containing two or more polypeptides of SEQ ID NO: 38 (wherein each X in the sequence table is independently any amino acid residue).
The invention also relates to the foregoing filamentous bacteriophage libraries wherein each X is independently an amino acid residue selected from the group consisting of A, C, D, E, F, G, H, I, K, L, M, N, P, Q, R, S, T, V, W and Y.
The invention also relates to a phage display method which uses the above-mentioned filamentous bacteriophage.
The invention also relates to the above phage display method, wherein the filamentous bacteriophage is of at least one type selected from the group consisting of fl phages, fd phages and M13 phages.
The invention also relates to the above phage display method, wherein the filamentous bacteriophage is an M13 phage.
The invention also relates to a phage display method which uses the above filamentous phage library.
The invention also relates to the above phage display method, wherein the filamentous bacteriophage is of at least one type selected from the group consisting of fl phages, fd phages and M 13 phages.
The invention also relates to the above phage display method, wherein the filamentous bacteriophage is an M13 phage.

The phage display method of the invention is able not only to efficiently produce random peptides, but also to provide novel sources in peptide conformation.

### BRIEF DESCRIPTION OF THE DRAWINGS

FIG. 1 shows the amino acid sequence of pIII.
FIG. 2 shows the amino acid sequence of a mutant pIII protein in the M13yt6 phage. The double underlined portion indicates an amino acid sequence that was inserted in vivo by an insertion mutation using oligonucleotide #186.
FIG. 3 shows the amino acid sequence of a mutant pIII protein in the M13yt42 phage. The double underlined portion indicates the mutant pIII amino acid sequence obtained by cloning, at the EcoRV-KpnI restriction enzyme site on the M13yt6 phage, synthetic DNA prepared by annealing oligonucleotide #250 and oligonucleotide #251.
FIG. 4 shows the amino acid sequence of a mutant pIII protein in the M13yt27 phage. The double underlined portion indicates the mutant pIII amino acid sequence obtained by cloning, at the EcoRV-KpnI restriction site of the M13yt6 phage, synthetic DNA prepared by annealing oligonucleotide #155 and oligonucleotide #156. The boxed-in portion indicates the amino acid sequence of somatostatin-14.
FIG. 5 shows the amino acid sequence of a mutant pIII protein in the M13mk7 phage. The double underlined portion indicates the mutant pIII amino acid sequence obtained by cloning, at the EcoRV-KpnI restriction site of the M13yt6 phage, DNA prepared by annealing oligonucleotide #246 and oligonucleotide #247 then converting to double-stranded DNA with the klnow fragment of DNA polymerase. The boxed-in portion indicates the amino acid sequence of somatostatin-14.
FIG. 6 shows the phage-forming abilities of mutant phages.
FIG. 7 shows the identification of somatostatin-14 by Western blotting a fusion pIII protein.
FIG. 8 shows the identification of somatostatin-14 by ELISA assay on phage particles that cloned somatostatin-14.
FIG. 9 shows the structure of gapped DNA: oligonucleotide #255 - oligonucleotide #256 - oligonucleotide #257.
FIG. 10-1 shows the structures of a CKNFX₄FTSC library pIII fusion protein and the corresponding DNA. The underlined portion indicates the inserted peptide.
FIG. 10-2 shows the structures of a CKNFX₄FTSC library pIII fusion protein and the corresponding DNA. The underlined portion indicates the inserted peptide.
FIG. 11 is a plot of amino acid frequencies in the CKNFX₄FTSC library versus the reported amino acid frequencies in the Ph.D. 7 library.
FIG. 12 compares the amino acid frequencies predicted from codons with the measured amino acid frequencies.
FIG. 13 is a plot of the correlation between amino acid frequencies (%) for the random sequence portions in various random peptide libraries and the frequencies determined from theoretical values for the number of amino acid residues predicted from codons in the inserted oligonucleotides.
FIG. 14 shows the sequences of synthetic oligonucleotides.
FIG. 15 shows the binding curves (measured antibody titers) for mouse anti-M13mp18 antisera.

### BEST MODE FOR CARRYING OUT THE INVENTION

The filamentous bacteriophages that may be used in the present invention include fl phages, fd phages and M13 phages. Of these, the use of M13 phages is preferred.

The present invention is described below using specifically M13 phages. However, the invention is not limited to the use of M13 phages, the use of any filamentous bacteriophage being possible.

### (1) Searching for Sites in pIII Where Peptide Can be Fused, and Production of Cloning Vectors

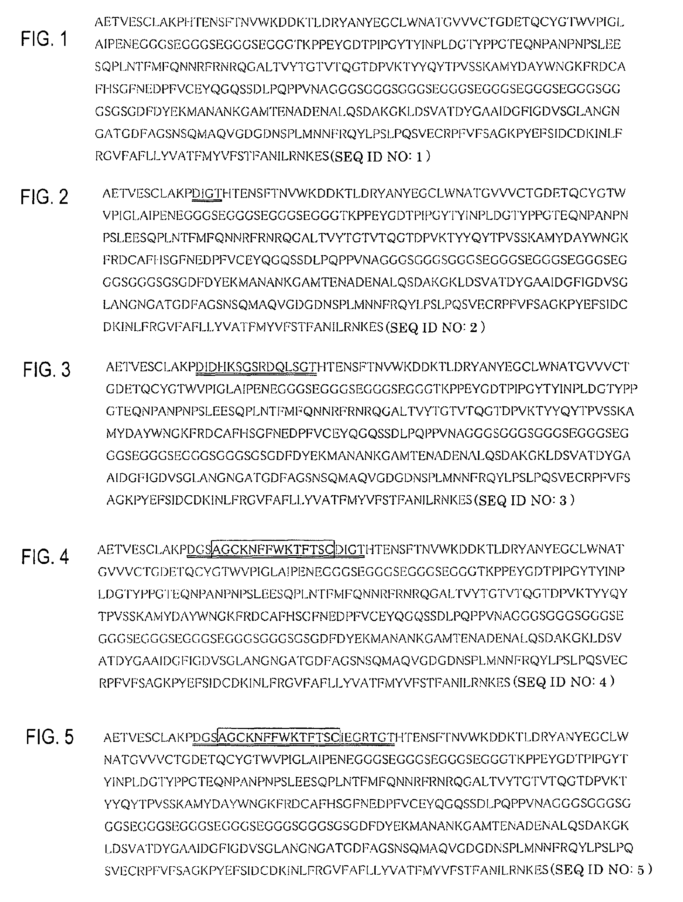

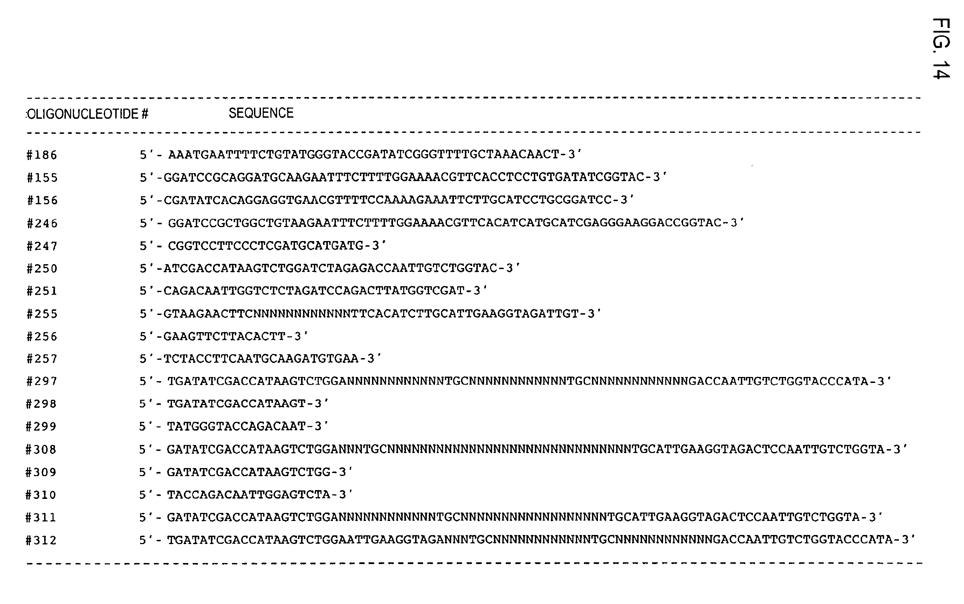

The inventor searched for sites where a peptide can be inserted at the interior of the pIII proteins in M13 phages. With regard to insertion sites, with M13KO7 phage ssDNA as the template, insertion mutations of gene-3 were induced in vitro using various oligonucleotides. The oligonucleotides were designed so as to enable the introduction of the four amino acid residues Asp-Ile-Gly-Thr at various insertion sites, and the introduction of a EcoRv-KpnI restriction enzyme site in the DNA. As a result, from mutations carried out in vitro using oligonucleotide #186 shown in FIG. 14, there was obtained a M13yt6 phage in which four amino acid residues were inserted between the proline residue at position 11 and the histidine residue at position 12. To facilitate cloning of the M13yt6 phage, DNA obtained by annealing two oligonucleotides (oligonucleotide #250 and oligonucleotide #251) was cloned at the EcoRV-KpnI restriction enzyme site to create the M13yt42 phage. FIGS. 1 to 3 show the amino acid sequences of pIII proteins from, respectively, an M13 phage, an M13yt6 phage, and an M13yt42 phage (SEQ ID NOS: 1 to 3).

DNA obtained by annealing two oligonucleotides (oligonucleotide #155 and oligonucleotide #156) was cloned at the EcoRV-KpnI restriction enzyme site of M13yt6 phage RF DNA, thereby creating a M13yt27 phage. FIG. 4 shows the amino acid sequence (SEQ ID NO: 4) for mutant pIII in the M13yt27 phage.

Also, DNA obtained by annealing two oligonucleotides (oligonucleotide #246 and oligonucleotide #247) and subsequent conversion to double-stranded DNA with the klnow fragment of DNA polymerase was cloned at the EcoRV-KpnI restriction enzyme site of the M13yt6 phage RF DNA to create the M13mk7 phage. FIG. 5 shows the amino acid sequence (SEQ ID NO: 5) for mutant pIII in the M13mk7 phage.

The mutant pIII proteins of the M13yt27 phage and the M13mk7 phage were obtained by the insertion of, respectively, 21 amino acid residues and 24 amino acid residues between the proline residue at position 11 and the histidine residue at position 12 of native pIII; both contain the amino acid sequence of somatostatin-14. It was thus found that either M13yt6 or M13yt42 can function as an M13 phage vector capable of inserting amino acid residues between the proline residue at position 11 and the histidine residue at position 12 of the pIII protein.

Cloning using the phage vector M13yt6 or M13yt42 involves inserting amino acid residues between the proline residue at position 11 and the histidine residue at position 12 of pIII. The pIII protein functions both in the infection of E. coli and in phage particle formation by interaction with the phage protein g6p, and the N1 domain of pIII is divided in two regions by cloning. This insertion site exists inside the C7-C36 disulfide bridge of the N1 domain. However, it has been reported that the C7-C36 disulfide linkage possesses a function essential to the formation of the N1 domain conformation; if this disulfide linkage is dissociated, a phage particle is not produced (Non-Patent Document 15).

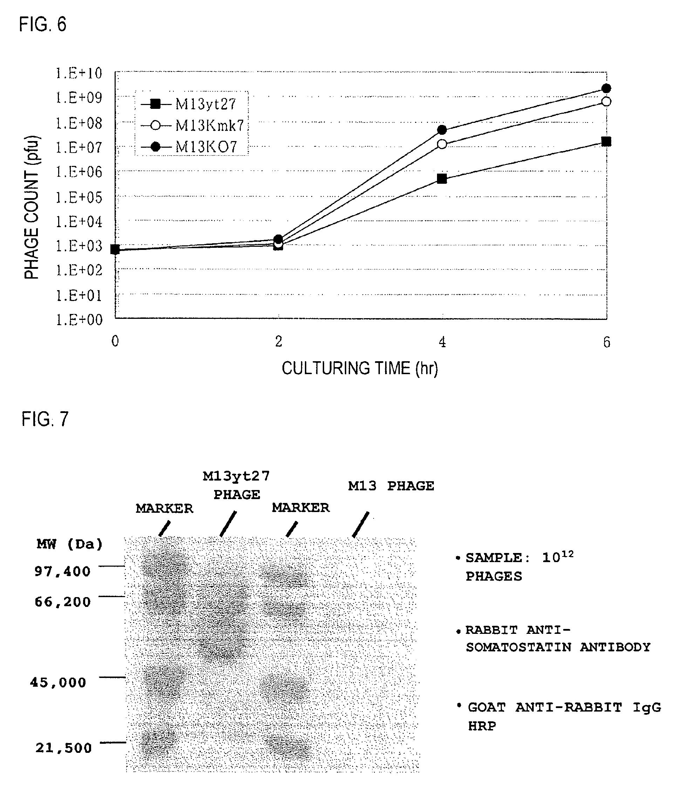

Hence, the influence on N1 domain function of inserting amino acid residues between the proline residue at position 11 and the histidine residue at position 12 of pIII was investigated. Using the M13yt27 phage and the M13mk7 phage, the phage-producing abilities of the respective mutant pIII proteins were measured. E. coli JM109 was infected with 500 pfu of, respectively, M13yt27 phage, M13mk7 phage and, as a control, M13KO7 phage, and the phage-producing abilities of each after 6 hours of culturing were measured. The results are shown in FIG. 6. The M13yt27 and M13mk7 phages containing mutant pIII proteins had phage-producing abilities 6 hours after phage infection which, in spite of being lower than the phage-producing ability of the M13KO7 phage serving as the control, were still sufficient. This indicates that the mutant pIII proteins of the M13yt27 phage and the M13mk7 phage retained an infecting ability in phage infection.

### (2) Phage Display of Somatostatin-14

In the M13yt27 phage, DNA coding for somatostatin-14 is fused with gene-3. The fact that the M13yt27 phage has a somatostatin-14 peptide sequence on the pIII proteins was verified by Western blotting using an anti-somatostatin antibody. FIG. 7 shows the results obtained by isolating the total protein of the M13yt27 phage by SDS-PAGE, and carrying out Western blotting using an anti-somatostatin antibody. In the M13yt27 phage, the approximately 52 kDa band was stained with the anti-somatostatin antibody. This molecular size agrees with the molecular size of the pIII proteins (Goldsmith, M.E. and Konigsberg, W.H. (1977): "Adsorption protein of the bacteriophage fd: Isolation, molecular properties, and location in the virus," Biochemistry 16, 2686-94). These results indicate that the pIII on the phage exists as a fusion protein of somatostatin-14.

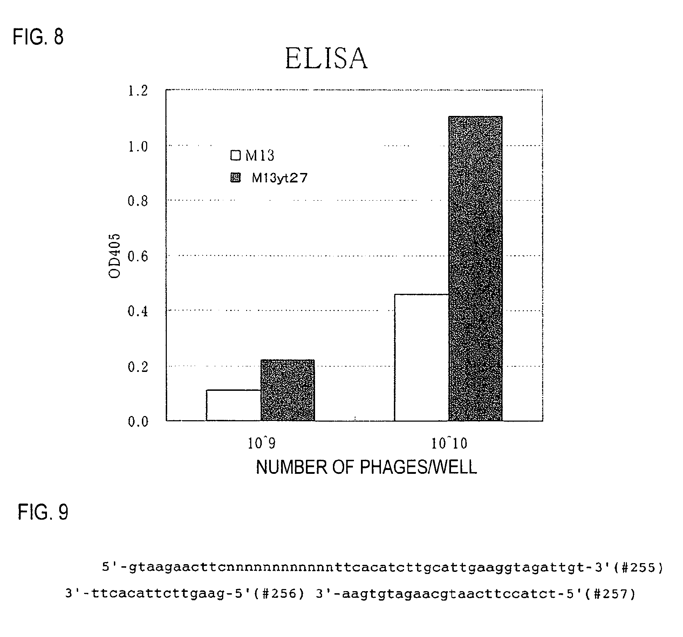

To determine whether somatostatin-14 display on M13yt27 phage particles functions in screening methods such as panning, ELISA assays using anti-somatostatin antibody were carried out for M13yt27 phage. The results are shown in FIG. 8. In titration up to 1×10⁸ phages/well, a significant difference is not observable between the M13yt27 phage and the M13KO7 phage. However, at 1×10⁹ phages/well and 1×10¹⁰ phages/well, M13yt27 phage was found to bind significantly to the anti-somatostatin antibody compared with M13KO7 phage. This indicates that somatostatin-14 is displayed on the M13yt27 phage, and that the peptide sequence of somatostatin-14 reacts with the anti-somatostatin antibody.

### (3) Construction and Evaluation of Peptide Libraries

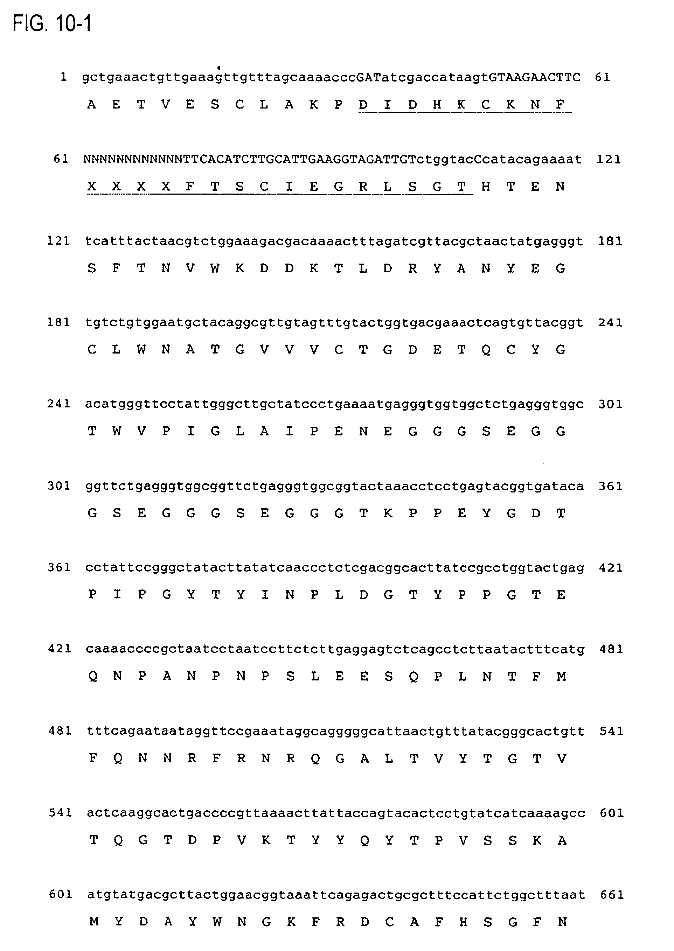

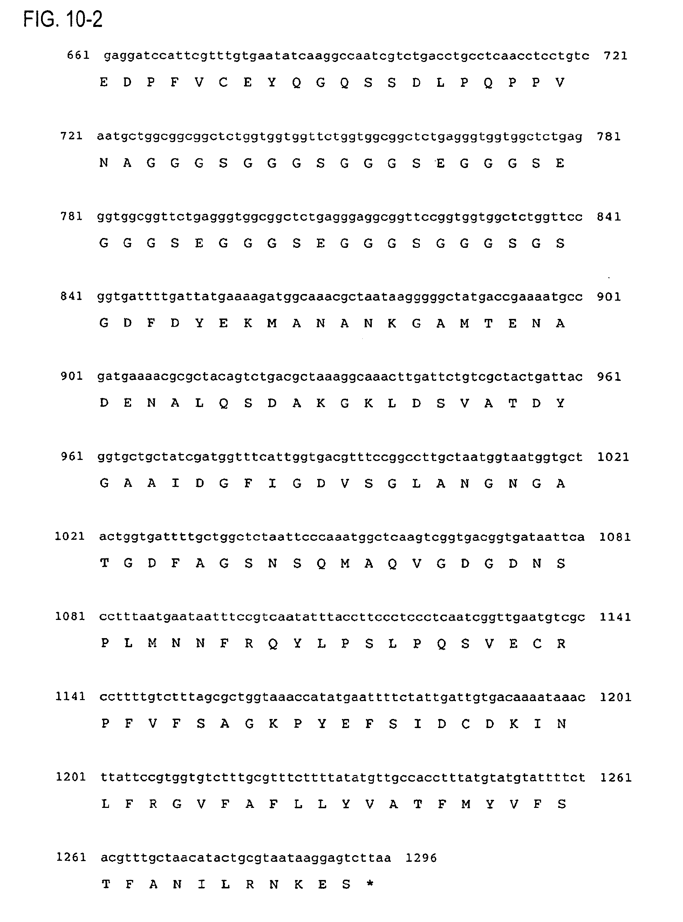

Peptide libraries were constructed by inserting peptides having random sequences between the proline residue at position 11 and the histidine residue at position 12 of the pIII protein. First, random tetrapeptides were produced using a peptide scaffold of somatostatin-14. Somatostatin-14 is a peptide hormone which forms the amino acid sequence AGCKNFFWKTFTSC and disulfide linkages within the molecule, and regulates the secretion of hormones such as growth hormone, glu-cagons, insulin and gastrin. The four amino acid residues FWKT located at the center of the loop structure are replaced with the random amino acid sequences. DNA having the random sequences was cloned at the BstXI restriction enzyme site of the M13yt42 phage vector. Gapped DNA like that shown in FIG. 9 which was obtained by annealing three oligonucleotides (oligonucleotide #255, oligonucleotide #256 and oligonucleotide #257) was used as the insertion DNA containing the random sequences. FIGS. 10-1 and 10-2 show the amino acid sequences, and the corresponding DNA sequences, of the N1 domains in the fusion proteins of pIII and random peptides obtained by cloning. Through such cloning, 1.0×10⁶ pfu independent clones were obtained. The theoretical number of different random peptides is 20⁴, that is, 1.6×10⁵ types. Hence, the experimentally obtained library size of 1.0×10⁶ types of clones exceeded the theoretical value for random peptides.

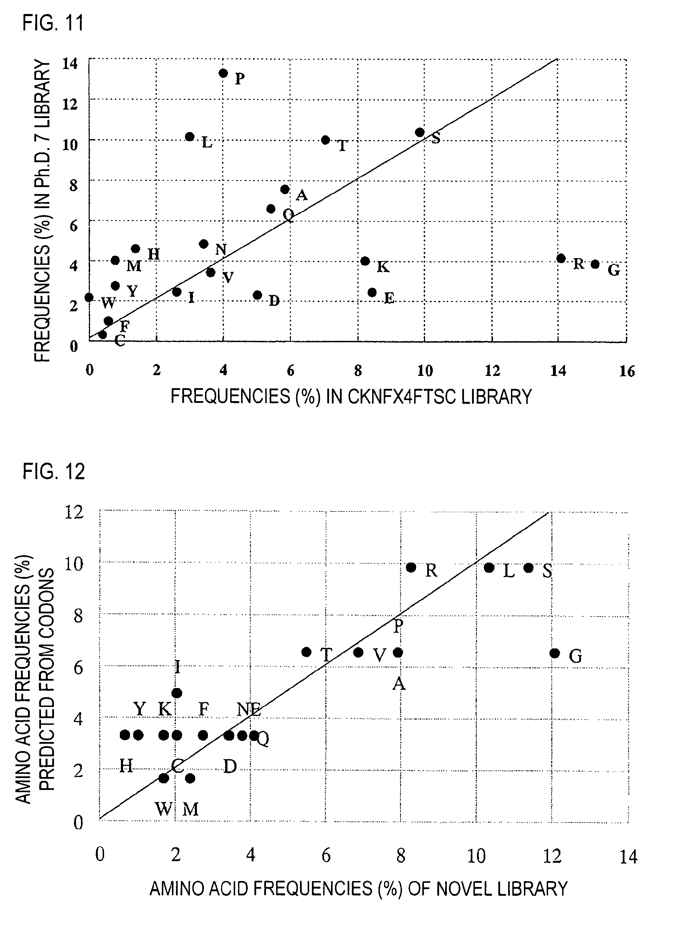

With regard to the assessment of random peptide libraries, the enormous number of random peptides precludes analysis of all the clones, although a method has been reported for assessing libraries based on statistics for the amino acid sequences of a portion of the clones (Rodi, D.J., Soares, A.S. and Makowski, L. (2002): "Quantitative assessment of peptide sequence diversity in M13 combinatorial peptide phage display libraries," J. Mol. Biol. 322, 1039-52). Based on this method, an understanding of the properties of the CKNFX₄FTSC library was achieved. The amino acid sequences of the random sequence portions from 124 clones appropriately selected from the 1.0×10⁶ types of clones were determined from the base sequences of the corresponding DNA portions. The amino acid frequencies at each position and for all positions on the random amino acid sequences were added up. Those results are shown in Table 1. The CKNFX₄FTSC library is characterized in that the glycine residue frequency in the random sequences of four peptides was highest at 15.7%, and the proline residue frequency was 3.9%. By contrast, in the already existing Ph.D. 7 library, which is a library of cyclic random heptapeptides fused at the N-terminus of pIII, in the random sequences of seven peptides, proline residues were most common at 13.3%, and the frequency of glycine residues was 3.9% (Rodi et al. (2002): J. Mol. Biol. 322, 1039-52). FIG. 11 shows the amino acid frequencies in the random sequences for these libraries. These results show that, in terms of functional diversity (Rodi et al. (2002): J. Mol. Biol. 322, 1039-52), the CKNFX₄FTSC library is a glycine-rich library, which is quite different from the existing pIII N-terminal fused libraries that are proline-rich.

**Table 1. Frequencies of amino acid residues in 124 clones randomly selected from CKNFX₄FTSC library. The position numbers in the random domain are assigned from the N-terminus.**

| Amino acid | Position 1 | Position 2 | Position 3 | Position 4 | Total | Percent (%) |
|---|---|---|---|---|---|---|
| A | 4 | 6 | 9 | 10 | 29 | 5.8 |
| C | 0 | 0 | 1 | 1 | 2 | 0.4 |
| D | 5 | 7 | 7 | 6 | 25 | 5.0 |
| E | 8 | 11 | 13 | 10 | 42 | 8.5 |
| F | 1 | 1 | 0 | 1 | 3 | 0.6 |
| G | 17 | 27 | 11 | 20 | 75 | 15.1 |
| H | 2 | 3 | 0 | 2 | 7 | 1.4 |
| I | 5 | 2 | 3 | 3 | 13 | 2.6 |
| K | 12 | 10 | 10 | 9 | 41 | 8.3 |
| L | 7 | 0 | 4 | 4 | 15 | 3.0 |
| M | 1 | 1 | | 1 | 4 | 0.8 |
| N | 6 | 2 | 4 | 5 | 17 | 3.4 |
| P | 4 | 4 | 9 | 3 | 20 | 4.0 |
| Q | 8 | 5 | 7 | 7 | 27 | 5.4 |
| R | 20 | 19 | 13 | 18 | 70 | 14.1 |
| S | 6 | 16 | 13 | 14 | 49 | 9.9 |
| T | 11 | 7 | 10 | 7 | 35 | 7.1 |
| V | 4 | 3 | 8 | 3 | 18 | 3.6 |
| W | 0 | 0 | 0 | 0 | 0 | 0.0 |
| Y | 3 | 0 | 1 | 0 | 4 | 0.8 |

The amino acid frequencies in the random sequences, as predicted from oligonucleotide codons in cloning, are defined as the technical diversity; if there is no biological bias after cloning, the amino acid frequencies from the codons should be similar to the measured frequencies. Codon predictions were arrived at by calculating the frequency information as percentages (%) from a 61 codon system. FIG. 12 compares the codon predicted frequencies with the measured amino acid frequencies in the CKNFX₄FTSC library. As a result, the correlation coefficient between the theoretical values and the measured values is r = 0.861, demonstrating a positive correlation therebetween. The correlation coefficient for the 20 amino acid residues other than glycine was r = 0.908, indicating a high positive correlation. These results mean that, in the cloning of random oligomers using the M13yt42 phage vector, there is little biological bias in such processes as pIII translocation and secretion.

Several libraries were constructed in order to investigate the permissibility of the M13yt42 phage vector in the diversity of peptide sizes or peptide designs that can be inserted. The libraries were all created by cloning random sequence-containing double-stranded DNA at the BstXI site of the M13yt42 vector. The double-stranded DNA to be inserted was prepared by carrying out, using random oligonucleotide as the template, a polymerase chain reaction (PCR) with two primers, then cleaving the resulting double-stranded DNA with the restriction enzyme BstXI. The combinations of oligonucleotides used in constructing each library were as follows: oligonucleotide #308, oligonucleotide #298 and oligonucleotide #310 were used to construct the XCX₁₀C library; oligonucleotide #311, oligonucleotide #298 and oligonucleotide #310 were used to construct the X₄CX₆C library; oligonucleotide #312, oligonucleotide #309 and oligonucleotide #299 were used to construct the XCX₄CX₄ library; and oligonucleotide #297, oligonucleotide #298 and oligonucleotide #299 were used to construct the X₄CX₄CX₄ library. In the construction of the XCX₁₀C library, the insertion of DNA fragments having a total length of 90 base pairs, corresponding to peptides of 30 amino acid residues, was possible. The amino acid sequences, and corresponding DNA sequences, of the mutant pIII proteins in these libraries are shown in SEQ ID NOS: 8 to 15. As a result, the M13yt42 phage vector was found to be capable of inserting peptides of sufficient length for creating a random peptide library of peptide-pIII fusion proteins, and capable of inserting disulfide cyclic peptides of diverse cyclic sizes.

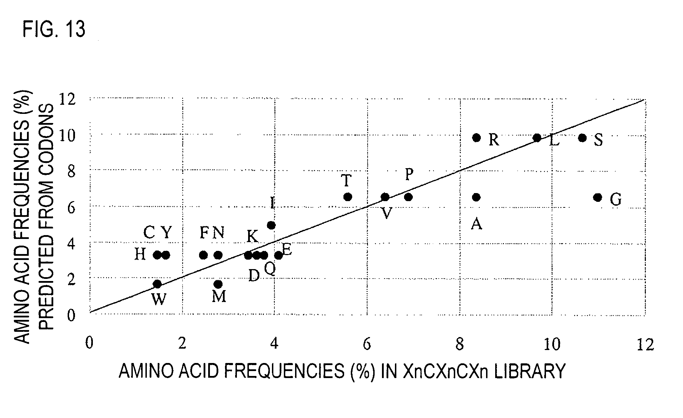

The amino acid sequences of the random sequence portions from 57 clones appropriately selected for the four libraries were determined from the base sequences of the corresponding DNA regions. Table 2 shows the results obtained from adding up the amino acid frequencies at all positions on the random amino acid sequences. The amino acid frequencies of the random sequence portions in these libraries were compared with the theoretical values for the amino acid frequencies predicted from the cloned DNA. Those results are shown in FIG. 13. In these libraries as well, just as in the CKNFX₄FTSC library, the glycine frequency was high, indicating glycine-rich library properties. The correlation coefficient between the measured and theoretical values for the amino acid frequencies was r = 0.891; hence, a high positive correlation existed between the two. Thus, the random peptide libraries obtained using the M13yt42 phage vector showed amino acid frequencies corresponding to the DNA to be cloned. This means that the cloning of random oligomers using the M13yt42 phage vector has little biological bias in such processes as pIII translocation and secretion in phage formation.

**Table 2. Numbers and frequencies (%) of amino acid residues in random sequence portions within each random peptide library. Codon predictions are percentages determined from the theoretical number of amino acid residues predicted from the inserted oligonucleotides.**

| Amino acid | X₄CX₄CX₄ | XCX₁₀C | XCX₄CX₄ | X₄CX₆C | Total | Frequency (%) | Codon prediction (%) |
|---|---|---|---|---|---|---|---|
| A | 13 | 11 | 16 | 11 | 51 | 8.36 | 6.56 |
| C | 4 | 4 | 2 | 0 | 10 | 1.64 | 3.28 |
| D | 10 | 9 | 1 | 2 | 22 | 3.61 | 3.28 |
| E | 10 | 10 | 1 | 4 | 25 | 4.10 | 3.28 |
| F | 2 | 5 | 5 | 3 | 15 | 2.46 | 3.28 |
| G | 15 | 17 | 20 | 15 | 67 | 10.98 | 6.56 |
| H | 4 | 1 | 1 | 3 | 9 | 1.48 | 3.28 |
| I | 10 | 7 | 4 | 3 | 24 | 3.93 | 4.92 |
| K | 14 | 3 | 4 | 2 | 23 | 3.77 | 3.28 |
| L | 13 | 23 | 17 | 6 | 59 | 9.67 | 9.84 |
| M | 5 | 4 | 5 | 3 | 17 | 2.79 | 1.64 |
| N | 5 | 8 | 1 | 3 | 17 | 2.79 | 3.28 |
| P | 12 | 10 | 8 | 12 | 42 | 6.89 | 6.56 |
| Q | 9 | 9 | 1 | 2 | 21 | 3.44 | 3.28 |
| R | 15 | 19 | 13 | 4 | 51 | 8.36 | 9.84 |
| S | 17 | 19 | 14 | 15 | 65 | 10.66 | 9.84 |
| T | 13 | 9 | 7 | 5 | 34 | 5.57 | 6.56 |
| V | 12 | 14 | 9 | 4 | 39 | 6.39 | 6.56 |
| W | 1 | 3 | 5 | 0 | 9 | 1.48 | 1.64 |
| Y | 6 | 2 | 1 | 1 | 10 | 1.64 | 3.28 |

### Example 1

### DNA Protocols Used in the Present Specification

Unless noted otherwise in the specification, the following protocols were used in carrying out the experiments. The culturing of M13 phages, the preparation of M13 phages, the extraction of DNA from E. coli and the M13 phages, enzyme reactions using DNA and oligonucleotides, and the transformation of E. coli were all carried out in accordance with the manufacturer's protocols for the materials used. In the absence of manufacturer's protocols, the protocols described in Molecular Cloning: A Laboratory Manual (Cold Spring Harbor Laboratory Press) were followed.

DNA base sequence determinations were carried out with special-purpose kit reagents using an ALF DNA sequencer (Amersham Bioscience) or an Open Gene DNA sequencer.

The oligonucleotides were custom synthesized by Greiner Japan KK and Genenet Co., Ltd. Base sequences for each oligonucleotide are shown in FIG. 14. The symbol # indicates the oligonucleotide number.

### Example 2

### Construction of the Vector M13yt6 for Expressing Random Peptides in a Filamentous Bacteriophage

Using the single-stranded DNA (abbreviated below as "ssDNA") of the M13KO7 phage, the oligonucleotide #186 shown in FIG. 14, and a Mutant K kit (Takara 6060; Takara Bio), mutation was carried out in vitro in accordance with the kit protocol. A phage solution, RF DNA and ssDNA were prepared from single plaques obtained by transformation. Restriction enzyme cleavage of the RF DNA at KpnI or EcoRV resulted in the identification of 8.7 Kb DNA fragments in each case. Restriction enzyme cleavage at two places, i.e., KpnI and HindIII or EcoRV and HindIII, resulted in the identification of DNA fragments of 5.7 Kb and 3.0 Kb in each case. In addition, the DNA base sequence at the mutation site in ssDNA was determined, and the M13yt6 phage thereby identified.

### Example 3

### Preparation of M13yt6 Vector DNA Fragments Double-Cleaved by Restriction Enzymes EcoRv and KpnI

The M13yt6 vector was cleaved by the restriction enzyme EcoRV (Takara Bio), then cleaved by the restriction enzyme KpnI (Takara Bio). The resulting 8.7 Kb EcoRV-KpnI double-cleaved DNA fragments were isolated by electrophoresis using 0.8% agarose gel, following which the DNA fragments were collected from the agarose gel. The concentration of the collected DNA was determined by comparing the degree of color development induced by 0.5 µg/ml of ethidium bromide with that of control DNA (λ HindIII marker: TOYOBO).

### Example 4

### Preparation of M13yt27 (Phage Capable of Displaying Somatostatin-14 Peptide)

DNA coding for the somatostatin-14 peptide was prepared from synthetic oligonucleotides. Specifically, the two oligonucleotides (oligonucleotide # 155 and oligonucleotide # 156 shown in FIG. 14) were each phosphorylated by T4 polynucleotide kinase. The reaction solutions were mixed in equal molar amounts, heated at 65°C for 30 minutes, then allowed to cool to room temperature, thereby giving a somatostatin-14 DNA solution. EcoRV-KpnI double-cleaved DNA fragments (8.7 Kb) from the M13yt6 vector (see Example 3) and the somatostatin-14 DNA were furnished to a ligase reaction. E. coli MV1184 was transformed in the ligase reaction solution. The single plaques that formed were screened, and plaques having somatostatin-14 DNA inserted between the EcoRV and KpnI restriction enzyme sites in M13yt6 were selected. A DNA fragment about 8.8 Kb in size was confirmed from the restriction enzyme BamHI cleavage of RF DNA prepared from these selected plaques, and was named M13yt27. The M13yt27 phage was identified by using the ssDNA of the M13yt27 phage to determine the DNA base sequence.

### Example 5

### Display of Somatostatin-14 Peptide by Western Blotting of M13yt27 Phage

Purified M13yt27 phage and purified M13KO7 phage (negative control) in amounts of 10⁹ pfu each were furnished to SDS-PAGE. The isolated protein was transferred to a nitrocellulose filter. The filter was blocked with a PBS solution of 10% skim milk at 37°C for 30 minutes. The filter was then reacted with a 400-fold dilution of rabbit anti-somatostatin-14 antiserum (Cambridge Research Biochemicals; catalog No. CA-08-325) as the primary antibody. After washing, the filter was reacted with a 3,000-fold dilution of HRP-labeled goat anti-rabbit IgG (H+L) (Life Technologies, Inc.; catalog No. 3859SA) as the second antibody. After washing, color development was carried out with an HRP color development kit (Bio-Rad Laboratories). A distinct 52 kDa band appeared on protein from the M13yt27 phage. On the other hand, no colored band whatsoever was noted on protein from the M13KO7 phage serving as the negative control.

### Example 6

### Production of Mouse Anti-M13 Phage Antiserum

After preparing the M13mp18 phage according to a conventional method, the phage solution was furnished to Sephacryl S-1000 superfine gel filtration (Pharmacia), thereby preparing a PBS solution having a phage concentration of 10¹⁴ pfu/ml. Next, 200 µl of Freund's complete adjuvant (Funakoshi) was intimately mixed with 200 µl of the phage solution, then administered to the pleural cavity of Balb/c female 6-week-old mice (Charles River Laboratories Japan) and a first sensitization carried out. After carrying out a total of three sensitizations at two-week intervals, 30 µl of phage solution was intravenously injected as a booster. On day 3 after the boost, all the blood was collected and centrifuged, yielding a supernatant as the antiserum.

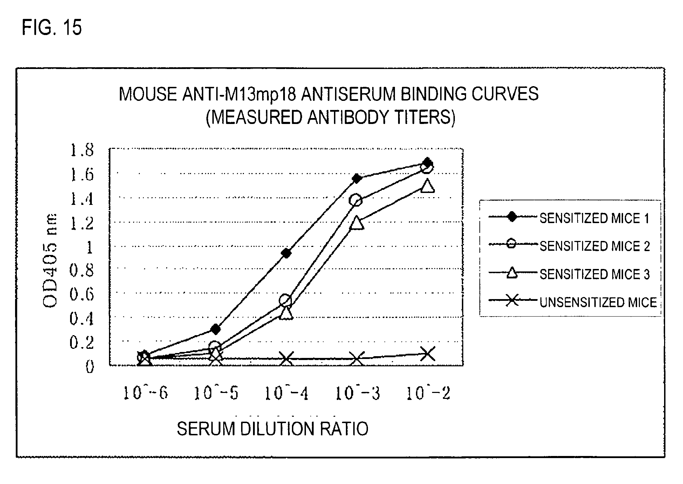

The properties of the antiserum were inspected by ELISA assay. First, 100 µl of a PBS solution having a phage concentration of 10¹⁴ pfu/ml was placed on a 96-well plate (Nunc MaxiSorp) and incubated at 37°C for 1 hour. The solution was removed and the plate was washed three times with PBS solution, following which 150 µl of a PBS solution containing 10% skim milk was added and the plate was incubated at 37°C for 1 hour. The solution was removed and the plate was washed with PBS solution, following which 100 µl of mouse serum solution diluted from 10² to 10⁶ fold with PBS solution containing 1% skim milk was added per well and the plate was incubated at 37°C for 1 hour. The solution was removed and the plate was washed three times with PBS solution, following which a 1% skim milk-containing PBS solution of 3,000-fold diluted HRP-labeled goat anti-mouse IgG (Bio-Rad Laboratories, catalog No. 170-6516) was added and the plate was incubated at 37°C for 1 hour. The solution was removed and the plate was washed three times with PBS solution containing 0.05% Tween-20, after which it was washed another two times with PBS solution and subsequently stained with an ABST color development kit (Bio-Rad Laboratories) according to the kit protocol, and the absorbance at 405 nm was measured. The results are shown in FIG. 15. Compared with a control serum, this antiserum showed, in ELISA assays, an ability to bind specifically to the M13mp18 phage at up to 10⁵ fold dilutions.

### Example 7

### Display of Somatostatin-14 Peptide by ELISA Assay of M13yt27 Phage

Rabbit anti-somatostatin-14 antiserum (Cambridge Research Biochemicals), 10 µg, was dissolved in 1 ml of coating buffer composed of 0.1 M NaHCO₃ (pH = 9.6), following which 100 µl of the solution was placed on a 96-well plate (Nunc MaxiSorp), and held at 4°C for 12 hours. The solution was then removed and the plate was washed three times with PBS solution, following which 150 µl of PBS solution containing 10% skim milk (blocking solution) was added and the mixture was incubated at 37°C for 1 hour. The solution was then removed and the plate was washed with a PBS solution containing 0.05% Tween-20, following which a purified phage solution of M13yt27 or M13KO7 (negative control) was added in an amount of from 10⁷ to 10¹⁰ pfu/well and incubated at 37°C for 1 hour. The solution was removed and the plate was washed three times with PBS solution, following which a 1% skim milk-containing PBS solution of 400-fold diluted mouse anti-M13 phage antiserum (see Example 6) was added as the primary antibody and the plate was incubated at 37°C for 1 hour. The solution was removed and the plate was washed three times with a PBS solution containing 0.05% Tween-20, following which a 1% skim milk-containing PBS solution of 3,000-fold diluted HRP-labeled goat anti-mouse IgG (Bio-Rad Laboratories, catalog No. 170-6516) was added as the secondary antibody and the solution was incubated at 37°C for 1 hour. The solution was removed, washed three times with a PBS solution containing 0.05% Tween-20, washed two more times with PBS solution, then stained using an ABST color development kit (Bio-Rad Laboratories) according to the kit protocol, and the absorbance at 405 nm was measured. The results are shown in FIG. 8. The amount of binding by the M13yt27 phage to the rabbit anti-somatostatin-14 antiserum exhibited a phage pfu volume-dependent increase.

### Example 8

### Production of Phage Vector M13yt42 by Insertion of BstXI Cloning Site in M13yt6 Phage

Two oligonucleotides (oligonucleotide #250 and oligonucleotide #251) were each phosphorylated by T4 polynucleotide kinase. The reaction solutions were mixed in equal amounts, heated at 65°C for 30 minutes, then allowed to cool to room temperature, thereby giving an insertion DNA fragment. An 8.7 Kb EcoRv-KpnI double-cleaved DNA fragment of M13yt6 (see Example 3) and the insertion DNA fragment were subjected to a ligase reaction, the resulting reaction solution was used to transform E. coli JM109, and plaques were formed in top agar using E. coli JM109 as the indicator strain. Single plaques were cultured and the phage prepared, following which RF DNA was collected from the phage particles. The RF DNA was cleaved by the restriction enzymes BstXI and XbaI, and a clone exhibiting the 8.7 Kb DNA fragment was selected. ssDNA was prepared from the clone and the DNA base sequence of the ssDNA was determined, thereby identifying the M13yt42 phage.

### Example 9

### Construction of Phage Library CKNFX₄FTSC of DNA Coding for Random Peptides Inserted in M13yt42 Phage Vector

Three oligonucleotides (oligonucleotide #255, oligonucleotide #256, and oligonucleotide #257) were each phosphorylated by T4 polynucleotide kinase. The reaction solutions were mixed in equal molar amounts, following which the mixture was heated at 65°C for 15 minutes, then held at 37°C (room temperature) for 15 minutes and treated at 4°C for 10 minutes to give insertion DNA fragments. M13yt42 RF DNA was cleaved by the restriction enzyme BstXI and isolated by electrophoresis using 0.8% agarose gel, following which 8.7 Kb DNA fragments were collected from the gel and used as the vector DNA. The 8.7 Kb DNA fragments and insertion DNA fragments were furnished to ligase reactions, and the resulting reaction solution was used to transform E. coli MC1061 by electroporation, thereby producing plaques in which E. coli JM109 serves as the indicator strain. The phage solution was recovered from the plate, thereby giving the phage library CKNFX₄FTSC.

### Example 10

### Transformation of E. coli by Electroporation

A single E. coli colony was subjected to a final liquid culture with 4 ml of SOB medium at 37°C. A final liquid culture solution (4 ml) was added to 400 ml of an SOB medium, and cultured at 37°C for 3 hours. When the cell solution reached an optical density at 620 nm of 0.51, the culture broth was cooled to 4°C. The cells were collected at 4,500 rpm for 15 minutes at 4°C, and the supernatant was discarded. The cells were then suspended in 400 ml of sterilized water, then collected at 4,500 rpm for 15 minutes at 4°C, and the supernatant was discarded. The cells were then suspended in 200 ml of sterilized water, collected at 4,500 rpm for 15 minutes at 4°C, and the supernatant was discarded. The cells were then suspended in 100 ml of sterilized water, collected at 4,500 rpm for 15 minutes at 4°C, and the supernatant was discarded. Next, the cells were suspended in 40 ml of a 10% glycerol solution, then collected at 4,500 rpm for 15 minutes at 4°C, and the supernatant was discarded. The cells were then suspended in 14 ml of a 10% glycerol solution, then collected at 4,500 rpm for 15 minutes at 4°C, and the supernatant was discarded. Next, the cells were suspended in 4 ml of a 10% glycerol solution, then dispensed in amounts of 200 µl, frozen on dry ice to give competent cells, and stored at -80°C.

The 200 µl portions of competent cells were thawed at 4°C and transferred to 0.2 cm electroporation cuvettes (Bio-Rad Laboratories). Next, 1 µl of ligase reaction solution was intimately mixed with the competent cells, and 2.5 kV, 400 Ω, 25 µF pulses were applied with a Gene Pulser (Bio-Rad Laboratories) for transferring genes. During plaque production, the transforming solution was diluted with medium, 0.2 ml of indicator strain and 0.5 ml of H top agar were added per 0.1 ml of the dilution, and the resulting mixture was seeded onto H agar plates and final liquid cultured at 37°C. In colony formation, 1 ml of SOC medium was added to the transforming solution, and culturing was carried out at 37°C for 1 hour. An appropriate amount of the culture was then seeded onto an LB agar plate and final liquid cultured at 37°C.

### Example 11

### Construction of Phage Library CKNFX₄FTSC of DNA Coding for Random Peptides Inserted in M13yt42 Phage Vector

Three oligonucleotides (oligonucleotide #255, oligonucleotide #256, and oligonucleotide #257) were each phosphorylated by T4 polynucleotide kinase. The reaction solutions were mixed in equal molar amounts, following which the mixture was heated at 65°C for 15 minutes, then held at 37°C (room temperature) for 15 minutes and treated at 4°C for 10 minutes to give insertion DNA fragments. M13yt42 RF DNA was cleaved by the restriction enzyme BstXI and isolated by electrophoresis using 0.8% agarose gel, following which 8.7 Kb DNA fragments were collected from the gel and used as the vector DNA. BstXI-cleaved DNA fragments of M13yt42 and insertion DNA fragments were subjected to a ligase reaction in a 1:4 molar ratio. E. coli JM109 was transformed by the calcium method in the reaction solution, producing plaques in which E. coli JM109 served as the indicator strain. The titer per transformation was 148,200. Transformation was repeated, thereby constructing the phage library CKNFX₄FTSC.

### Example 12

Construction of Phage Library X₄CX₄CX₄ of DNA Coding for Random Peptides Inserted in M13yt42 Phage Vector Three oligonucleotides (oligonucleotide #297, oligonucleotide #298, and oligonucleotide #299) were subjected to 25 cycles of polymerase chain reactions (PCR) with Taq DNA polymerase (each cycle consisting of 30 seconds at 94°C, 1 minute at 55°C and 1 minute at 75°C), followed by a final PCR reaction at 72°C for 10 minutes. The PCR product was cleaved by the restriction enzyme BstXI. The reaction solution was isolated with 4% to 20% polyacrylamide gel (precast gel, available from Daiichi Pure Chemicals), following which the 65 bp DNA fragments were collected and used as the insertion DNA fragments. M13yt42 RF DNA was cleaved by the restriction enzyme BstXI, and the 8.7 Kb DNA fragments were isolated by electrophoresis using an 0.8% agarose gel, then recovered from the gel to give vector DNA. The BstXI-cleaved DNA fragments of M13yt42 and the insertion DNA fragments were subjected to a ligase reaction in a 1:4 molar ratio. E. coli JM109 was transformed by the calcium method in the reaction solution, producing plaques in which E. coli JM109 served as the indicator strain. The phage solution was recovered from the plate, and rendered into the phage library X₄CX₄CX₄.

### Example 13

### Construction of Phage Library XCX₁₀C of DNA Coding for Random Peptides Inserted in M13yt42 Phage Vector

Three oligonucleotides (oligonucleotide #308, oligonucleotide #298, and oligonucleotide #310) were subjected to 25 cycles of polymerase chain reactions with Taq DNA polymerase (each cycle consisting of 30 seconds at 94°C, 1 minute at 55°C and 1 minute at 75°C), followed by a final PCR reaction at 72°C for 10 minutes. The PCR product was cleaved by the restriction enzyme BstXI. The reaction solution was isolated with 4% to 20% polyacrylamide gel (precast gel, available from Daiichi Pure Chemicals), following which the 65 bp DNA fragments were collected and used as the insertion DNA fragments. M13yt42 RF DNA was cleaved by the restriction enzyme BstXI, and the 8.7 Kb DNA fragments were isolated by electrophoresis using an 0.8% agarose gel, then recovered from the gel to give vector DNA. The BstXI-cleaved DNA fragments of M13yt42 and the insertion DNA fragments were subjected to a ligase reaction in a 1:4 molar ratio. E. coli JM109 was transformed by the calcium method in the reaction solution, producing plaques in which E. coli JM109 served as the indicator strain. The phage solution was recovered from the plate, and rendered into the phage library XCX₁₀C.

### Example 14

### Construction of Phage Library X₄CX₆C of DNA Coding for Random Peptides Inserted in M13yt42 Phage Vector

Three oligonucleotides (oligonucleotide #311, oligonucleotide #298, and oligonucleotide #310) were subjected to 25 cycles of polymerase chain reactions with Taq DNA polymerase (each cycle consisting of 30 seconds at 94°C, 1 minute at 55°C and 1 minute at 75°C), followed by a final PCR reaction at 72°C for 10 minutes. The PCR product was cleaved by the restriction enzyme BstXI. The reaction solution was isolated with 4% to 20% polyacrylamide gel (precast gel, available from Daiichi Pure Chemicals), following which the 65 bp DNA fragments were collected and used as the insertion DNA fragments. M13yt42 RF DNA was cleaved by the restriction enzyme BstXI, and the 8.7 Kb DNA fragments were isolated by electrophoresis using an 0.8% agarose gel, then recovered from the gel to give vector DNA. The BstXI-cleaved DNA fragments of M13KO7yt42 and the insertion DNA fragments were subjected to a ligase reaction in a 1:4 molar ratio. E. coli JM109 was transformed by the calcium method in the reaction solution, producing plaques in which E. coli JM109 served as the indicator strain. The phage solution was recovered from the plate, and rendered into the phage library X₄CX₆C.

### Example 15

### Construction of Phage Library XCX₄CX₄ of DNA Coding for Random Peptides Inserted in M13yt42 Phage Vector

Three oligonucleotides (oligonucleotide #312, oligonucleotide #309, and oligonucleotide #299) were subjected to 25 cycles of polymerase chain reactions with Taq DNA polymerase (each cycle consisting of 30 seconds at 94°C, 1 minute at 55°C and 1 minute at 75°C), followed by a final PCR reaction at 72°C for 10 minutes. The PCR product was cleaved by the restriction enzyme BstXI. The reaction solution was isolated with 4% to 20% polyacrylamide gel (precast gel, available from Daiichi Pure Chemicals), following which the 87 bp DNA fragments were collected and used as the insertion DNA fragments. M13yt42 RF DNA was cleaved by the restriction enzyme BstXI, and the 8.7 Kb DNA fragments were isolated by electrophoresis using an 0.8% agarose gel, then recovered from the gel to give vector DNA. The BstXI-cleaved DNA fragments of M13yt42 and the insertion DNA fragments were subjected to a ligase reaction in a 1:4 molar ratio. E. coli JM109 was transformed by the calcium method in the reaction solution, producing plaques in which E. coli JM109 served as the indicator strain. The phage solution was recovered from the plate, and rendered into the phage library XCX₄CX₄.

### Identification of Phage Libraries

RF DNA and ssDNA were extracted from single plaques following transformation. The RF DNA and the restriction enzyme-cleaved DNA were subjected to 0.8% agarose electrophoresis, and the DNA sizes identified. The DNA base sequence of the ssDNA was determined, and was identified as containing the desired DNA sequence. Treating the phage libraries as mixed clones, the preserved phage solution was amplified, and the DNA sizes for the RF DNA and for the restriction enzyme-cleaved DNA thereof were identified by 0.8% agarose electrophoresis.

## Claims

1. A mutant pIII protein having an amino acid residue inserted between a proline residue at position 11 and a histidine residue at position 12 of a filamentous bacteriophage pIII protein of SEQ ID NO: 1.

2. The mutant pIII protein of claim 1, wherein from 1 to 50 amino acid residues are inserted.

3. The mutant pIII protein of claim 1, wherein from 4 to 30 amino acid residues are inserted.

4. A polypeptide of SEQ ID NO: 34, 35, 36, 37 or 38 (wherein each X in the sequence table is independently any amino acid residue).

5. A polypeptide of SEQ ID NO: 2, 3, 4 or 5.

6. A polynucleotide which codes for the mutant pIII protein of any one of claims 1 to 3.

7. A polynucleotide which codes for the polypeptide of claim 4 or 5.

8. A vector containing the polynucleotide of claim 6 or 7.

9. The vector of claim 8, wherein the vector is a filamentous bacteriophage.

10. A host cell containing the vector of claim 8 or 9.

11. The host cell of claim 10, wherein the host cell is Escherichia coli.

12. A polypeptide library containing two or more polypeptides of SEQ ID NO: 34, 35, 36, 37 or 38 (wherein each X in the sequence table is independently any amino acid residue).

13. A filamentous bacteriophage containing the polynucleotide of claim 6 or 7.

14. A filamentous bacteriophage which displays the mutant pIII protein of any one of claims 1 to 3.

15. A filamentous bacteriophage which displays the polypeptide of claim 4 or 5.

16. The filamentous bacteriophage of any one of claims 13 to 15, wherein the filamentous bacteriophage is an M13 phage.

17. A filamentous bacteriophage library which displays two or more mutant pIII proteins of any one of claims 1 to 3.

18. A filamentous bacteriophage library which displays two or more polypeptides of SEQ ID NO: 34, 35, 36, 37 or 38 (wherein each X in the sequence table is independently any amino acid residue).

19. A phage display method, comprising using the filamentous bacteriophage of any one of claims 13 to 16.

20. The phage display method of claim 19, wherein the filamentous bacteriophage is an M13 phage.

21. A phage display method, comprising using the filamentous phage library of claim 18.

22. The phage display method of claim 21, wherein the filamentous bacteriophage is an M13 phage.
